# FASCICULE DE BREVET EUROPEEN

(11) **EP 3 452 489 B1**
(45) Date de publication et mention de la délivrance du brevet: **13.05.2020**
(21) Numéro de dépôt: 17719289.5
(22) Date de dépôt: 27.04.2017
(51) Int. Cl.: C07F 9/6584, A61P 25/24, A61K 31/675

(54) **NOUVEAUX DÉRIVÉS DE PHOSPHINOLACTONES ET LEURS UTILISATIONS PHARMACEUTIQUES**
NEUARTIGE PHOSPHINOLACTONDERIVATE UND PHARMAZEUTISCHE VERWENDUNGEN DAVON
NOVEL PHOSPHINOLACTONE DERIVATIVES AND PHARMACEUTICAL USES THEREOF

(30) Priorité: 02.05.2016 FR 1653960
(43) Date de publication de la demande: 13.03.2019
(73) Titulaire: Institut National de la Santé et de la Recherche Médicale (INSERM), 75013 Paris (FR); Ecole Pratique des Hautes Etudes, 75014 Paris (FR); Ecole Nationale Supérieure de Chimie de Montpellier, 34296 Montpellier Cedex 5 (FR); Centre National de la Recherche Scientifique, 75016 Paris (FR); Université de Montpellier, 34090 Montpellier (FR)
(72) Inventeur: MAURICE, Tangui, 34980 SAINT-GÉLY-DU-FESC (FR); VOLLE, Jean-Noël, 34830 JACOU (FR); VIRIEUX, David, 34000 MONTPELLIER (FR); PIRAT, Jean-Luc, 34130 SAINT-AUNES (FR); LABORDE, Coralie, 34560 POUSSAN (FR)
(74) Mandataire: Lavoix
(86) Numéro de dépôt international: PCT/EP2017/060129
(87) Numéro de publication internationale: WO 2017/191034

(56) Documents cités:
- WO-A2-2008/010985
- PIRAT J L ET AL: "Pallado-catalysed P-arylations and P-vinylation of 2-hydrogeno-2-oxo-1,4,2-oxazaphosphinanes" , TETRAHEDRON, ELSEVIER SCIENCE PUBLISHERS, AMSTERDAM, NL, vol. 61, no. 29, 18 juillet 2005 (2005-07-18), pages 7029-7036, XP027861194, ISSN: 0040-4020 [extrait le 2005-07-18]
- JEAN-NOËL VOLLE ET AL: "Drug discovery: phosphinolactone, in vivo bioisostere of the lactol group", ORGANIC & BIOMOLECULAR CHEMISTRY, vol. 8, no. 6, 1 janvier 2010 (2010-01-01) , page 1438, XP055332345, GB ISSN: 1477-0520, DOI: 10.1039/b919345f
- VOLLE J N ET AL: "Chiral phosphinyl analogues of 2-C-arylmorpholinols: 2-aryl-3,5-diphenyl-[1,4,2]-oxazaphosphina nes", TETRAHEDRON ASYMMETRY, PERGAMON PRESS LTD, OXFORD, GB, vol. 17, no. 9, 15 mai 2006 (2006-05-15), pages 1402-1408, XP024962074, ISSN: 0957-4166, DOI: 10.1016/J.TETASY.2006.05.003 [extrait le 2006-05-15]
- CRISTAU H J ET AL: "Synthesis, reactivity and stereochemistry of new phosphorus heterocycles with 5- or 6-membered rings", JOURNAL OF ORGANOMETALLIC CHEMISTRY, ELSEVIER-SEQUOIA S.A. LAUSANNE, CH, vol. 690, no. 10, 16 mai 2005 (2005-05-16) , pages 2472-2481, XP027708585, ISSN: 0022-328X [extrait le 2005-05-16]
- A. VAN DER LEE ET AL: "Structure-directing weak phosphoryl X H...O=P ( X = C, N) hydrogen bonds in cyclic oxazaphospholidines and oxazaphosphinanes", ACTA CRYSTALLOGRAPHICA. SECTION B, STRUCTURAL SCIENCE, vol. 64, no. 2, 14 mars 2008 (2008-03-14), pages 196-205, XP055332343, DK ISSN: 0108-7681, DOI: 10.1107/S0108768107061770
- Jean-Noël Volle ET AL: "Phosphono- and Phosphinolactones in the Life Sciences" In: "Advances in Heterocyclic Chemistry", 7 janvier 2016 (2016-01-07), Academic Press, XP055332311, ISSN: 0065-2725 vol. 118, pages 129-193, DOI: 10.1016/bs.aihch.2015.10.004, alinéa [05.7] composés 208,209
- JRME MONBRUN ET AL: "Diastereoselective Michael addition of 2-2-oxo-1,4,2-oxaza phosphinanes to olefins", TETRAHEDRON, ELSEVIER SCIENCE PUBLISHERS, AMSTERDAM, NL, vol. 67, no. 2, 26 octobre 2010 (2010-10-26), pages 540-545, XP028165023, ISSN: 0040-4020, DOI: 10.1016/J.TET.2010.10.078 [extrait le 2010-11-12]
- VOLLE ET AL: "Phosphinyl analogues of hydroxybupropion: (+/-)-2-aryl-3,3,5,5-tetramethyl-[1,4,2]-o xazaphosphinanes", TETRAHEDRON LETTERS, ELSEVIER, AMSTERDAM, NL, vol. 48, no. 27, 7 juin 2007 (2007-06-07), pages 4695-4697, XP022107524, ISSN: 0040-4039, DOI: 10.1016/J.TETLET.2007.05.014 cité dans la demande
- FAGADAR-COSMA ET AL: "Phosphorus compounds acting as plant hormones", ANNALS OF WEST UNIVERSITY OF TIMIoOARA. SERIES OF CHEMISTRY = ANALELE UNIVERSITÃ II DE VEST DIN TIMIoOARA. SERIA CHIMIE, UNIVERSITATEA DE VEST DIN TIMISOARA * FACULTATEA DE CHIMIE, BIOLOGIE, GEOGRAFIE , vol. 15, no. 2 1 janvier 2006 (2006-01-01), pages 151-158, XP008182743, ISSN: 1224-9513 Extrait de l'Internet: URL:http://www.chimie.uvt.ro/awut_sc/awut/ Z_Cont&Summary_Vol%2015%282%29%202006.pdf
- RENDIE LIU ET AL: "Efficient One-Pot Synthesis of Novel Spirooxindole-Fused Phosphorous Heterocycle Derivatives by a Three-Component Domino Reaction", HETEROATOM CHEMISTRY, vol. 25, no. 3, 21 mars 2014 (2014-03-21), pages 140-146, XP055332346, US ISSN: 1042-7163, DOI: 10.1002/hc.21146
- MUDARIS DIMUKHAMETOV ET AL: "Synthesis and Crystal Structure of 2-Substituted 3-Aryl-2-Oxophenylbenzo[ E ]-1,4,2-Oxazaphosphinanes", PHOSPHORUS, SULFUR AND SILICON AND THE RELATED ELEMENTS, vol. 190, no. 5-6, 3 juin 2015 (2015-06-03), pages 943-946, XP055332349, US ISSN: 1042-6507, DOI: 10.1080/10426507.2014.993756
- M N Dimukhametov ET AL: "A convenient synthesis and spatial structure of 22aryll22oxoo22phenylbenzo[e]]1,4,22oxazap hosphinanes*", Russian Chemical Bulletin International Edition, 1 janvier 2013 (2013-01-01), pages 1882-1891, XP055332351, Extrait de l'Internet: URL:http://rd.springer.com/content/pdf/10. 1007/s11172-013-0271-2.pdf [extrait le 2017-01-05]
- EUGENIA FAGADAR-COSMA ET AL: "Synthesis, characterization and correlative biological effects in wheat of a benzoxaza- and a diaza-phosphorus(V) heterocycles", JOURNAL OF THE SERBIAN CHEMICAL SOCIETY, vol. 71, no. 10, 1 janvier 2006 (2006-01-01), pages 1031-1038, XP055332339, Belgrade ISSN: 0352-5139, DOI: 10.2298/JSC0610031F
- JIA ZHOU ET AL: "Studies on Cyclic [alpha]-Aminoalkanephosphonate Compounds: A Novel Synthesis of 2-Phenyl-1,4,2-Benzoxaza (or diaza)phosphorin 2-Oxides", SYNTHESIS, vol. 1999, no. 1, 1 janvier 1999 (1999-01-01), pages 40-42, XP055332338, STUTTGART, DE. ISSN: 0039-7881, DOI: 10.1055/s-1999-3676

## Description

La présente invention a pour objet de nouveaux composés, dérivés de phosphinolactones, ainsi que leurs utilisations, notamment pharmaceutiques, et plus particulièrement pour le traitement de maladies neurodégénératives.

L'invention a également pour objet des compositions pharmaceutiques contenant lesdits composés.

A ce jour, on connait des analogues phosphinolactones de l'hydroxybupropion qui ont été synthétisés dans le but de développer de nouveaux antidépresseurs. Ces composés ont été testés sur des modèles animaux sur des réponses liées à la dépression et ont montré une activité d'antidépresseur significative, en diminuant le temps d'immobilité de souris soumises au test de nage forcée.

Les maladies neurodégénératives, telles que la maladie d'Alzheimer, la maladie de Parkinson, la maladie de Huntington ou encore la sclérose latérale amyotrophique sont des maladies chroniques invalidantes à évolution lente et discrète. Elles provoquent généralement une détérioration du fonctionnement des cellules nerveuses, en particulier les neurones, pouvant conduire à la mort cellulaire (ou neurodégénérescence). Les troubles induits par les maladies neurodégénératives sont variés et peuvent être d'ordre cognitivo-comportemental, sensoriel et moteur.

Au fur et à mesure que la recherche progresse, de nombreuses similitudes apparaissent reliant ces maladies les unes aux autres, surtout au niveau cellulaire notamment par l'agrégation de protéines atypiques et la mort neuronale induite. La découverte de ces similitudes offre l'espoir d'avancées thérapeutiques qui pourraient améliorer simultanément de nombreuses maladies.

La maladie d'Alzheimer est une maladie neurodégénérative à progression lente qui détériore de façon progressive les neurones dans les régions du cerveau impliquées dans la mémoire, l'apprentissage et le raisonnement. Elle est caractérisée par une accumulation extracellulaire de peptide β-amyloïde (Aβ), formant des plaques amyloïde dans le cerveau.

On estime à ce jour à environ 3% d'individus âgés entre 65 et 74 ans avec la maladie d'Alzheimer, et jusqu'à environ la moitié parmi les individus âgés de 85 ans et plus.

WO 2008/010985 A2 divulgue des composés organophosphorés cycliques destinés à être utilisés dans le traitement de maladies neurodégénératives.

Il existe donc à ce jour un besoin de disposer de composés efficaces pour traiter les maladies neurodégénératives, et notamment la maladie d'Alzheimer.

La présente invention a pour but de fournir de nouveaux composés particulièrement efficaces pour traiter les maladies neurodégénératives.

Ainsi, la présente invention concerne un composé de formule (I) suivante : 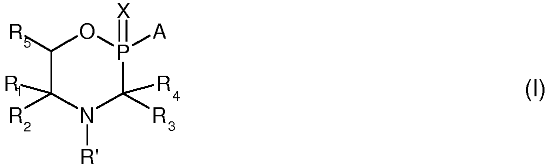 dans laquelle :
- X est O ou S ;
- A est choisi dans le groupe constitué :
   - des groupes (C₆-C₁₀)aryles,
   - des groupes hétéroaryles comprenant de 5 à 10 atomes, et
   - des hétérocycloalkyles comprenant de 5 à 10 atomes,
      lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ;
      Rₐ et R_{b}, identiques ou différents, étant H ou un groupe (C₁-C₆)alkyle ;
      R_{C} étant un radical -(C₁-C₆)alkylène-(C₆-C₁₀)aryle, notamment un radical -CH₂-(C₆-C₁₀)aryle ;
      R'ₐ étant choisi parmi les groupes CF₃, CHF₂ et CH₂F ;
- R' est H ou un groupe (C₁-C₆)alkyle ;
- R₁, R₂, R₃, R₄ et R₅, identiques ou différents, sont choisis dans le groupe constitué de :
   - H,
   - des groupes (C₁-C₆)alkyles,
   - des groupes (C₆-C₁₀)aryles,
      R₁ et R₂ pouvant former ensemble, avec l'atome de carbone qui les porte, un cycle spiranique comprenant de 3 à 6 atomes de carbone, et/ou R₃ et R₄ pouvant former ensemble, avec l'atome de carbone qui les porte, un cycle spiranique comprenant de 3 à 6 atomes de carbone,
      pour son utilisation pour le traitement de maladies neurodégénératives.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I), font également partie de l'invention.

Dans le cadre de la présente invention, on entend par Cₜ-C_{z} une chaîne carbonée pouvant avoir de t à z atomes de carbone.

Selon l'invention, le terme « atome d'halogène » désigne les atomes de fluor, chlore, brome ou iode.

Dans le cadre de la présente invention, on entend par « groupe alkyle » un groupe aliphatique hydrocarboné, saturé, linéaire ou ramifié comprenant, sauf mention contraire, de 1 à 6 atomes de carbone. A titre d'exemples, on peut citer les groupes méthyle, éthyle, n-propyle, isopropyle, butyle, isobutyle, tertbutyle ou pentyle.

Dans le cadre de la présente invention, on entend par « radical -(C₁-C₆)alkylène » un radical bivalent, linéaire ou ramifié, comprenant de 1 à 6 atomes de carbone, correspondant à un groupe alkyle avec un atome d'hydrogène en moins.

Dans le cadre de la présente invention, on entend par « groupe aryle » un groupe aromatique cyclique comprenant entre 6 et 10 atomes de carbone. A titre d'exemples de groupes aryles, on peut citer les groupes phényle ou naphtyle.

Dans le cadre de la présente invention, on entend par « groupe hétéroaryle » un groupe monocyclique ou bicyclique aromatique de 5 à 10 chaînons contenant de 1 à 4 hétéroatomes choisis parmi O, S ou N. A titre d'exemples, on peut citer les groupes imidazolyle, thiazolyle, oxazolyle, furanyle, thiophényle, pyrazolyle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle.

A titre d'hétéroaryle comprenant 5 à 6 atomes, dont 1 à 4 atomes d'azote, on peut notamment citer les groupes représentatifs suivants : pyrrolyle, pyrazolyle, 1,2,3-triazolyle, 1,2,4-triazolyle, tétrazolyle, 1,2,3-triazinyle.

On peut également citer, à titre d'hétéroaryle, le thiophényle, l'oxazolyle, le furazanyle, le 1,2,4-thiadiazolyle, le naphthyridinyle, le quinoxalinyle, le phtalazinyle, l'imidazo[1,2-a]pyridine, l'imidazo[2,1-b]thiazolyle, le cinnolinyle, le benzofurazanyle, l'azaindolyle, le benzimidazolyle, le benzothiophényle, le thiénopyridyle, le thiénopyrimidinyle, le pyrrolopyridyle, l'imidazopyridyle, le benzoazaindole, le 1,2,4-triazinyle, l'indolizinyle, l'isoxazolyle, l'isoquinolinyle, l'isothiazolyle, le purinyle, le quinazolinyle, le quinolinyle, l'isoquinolyle, le 1,3,4-thiadiazolyle, le thiazolyle, l'isothiazolyle, le carbazolyle, ainsi que les groupes correspondants issus de leur fusion ou de la fusion avec le noyau phényle.

Dans le cadre de la présente invention, on entend par « groupe hétérocycloalkyle » un groupe monocyclique ou bicyclique saturé ou partiellement insaturé de 5 à 10 chaînons, comprenant de un à trois hétéroatomes choisis parmi O, S ou N. Selon l'invention, le groupe hétérocycloalkyle est rattaché au reste de la molécule par un atome de carbone ou par un hétéroatome et le terme hétérocycloalkyle bicylique inclue les bicycles fusionnés et les cycles de type spiro.

A titre d'hétérocycloalkyle saturé comprenant de 5 à 6 atomes, on peut citer l'oxétanyle, le tétrahydrofuranyle, le dioxolanyle, le pyrrolidinyle, l'azépinyle, l'oxazépinyle, le pyrazolidinyle, l'imidazolidinyle, le tétrahydrothiophényle, le dithiolanyle, le thiazolidinyle, le tétrahydropyranyle, le tétrahydropyridinyle, le dioxanyle, le morpholinyle, le pipéridinyle, le pipérazinyle, le tétrahydrothiopyranyle, le dithianyle, le thiomorpholinyle, ou l'isoxazolidinyle.

Lorsque l'hétérocycloalkyle est substitué, la(les) substitution(s) peu(ven)t être sur un(des) atome(s) de carbone et/ou sur le(s) hétéroatome(s). Lorsque l'hétérocycloalkyle comporte plusieurs substituants, ceux-ci peuvent être portés par un même atome ou des atomes différents.

Les composés de formule (I) telle que définie ci-dessus sont utilisés pour le traitement de maladies neurodégénératives.

Dans le cadre de l'invention, le terme « maladie neurodégénérative » désigne une maladie qui est causée par une altération du système nerveux central et peut être identifiée par la mort neuronale. La mort des cellules neuronales observée dans les maladies neurodégénératives est souvent précédée d'un dysfonctionnement neuronal, parfois pendant plusieurs années.

Le terme « maladie neurodégénérative » inclut donc les pathologies ou les troubles caractérisés par un dysfonctionnement neuronal et éventuellement par la mort de cellules neuronales. A titre d'exemples de maladies neurodégénératives, on peut citer la démence associée au VIH, la sclérose en plaques, la maladie d'Alzheimer, la maladie de Parkinson, la sclérose latérale amyotrophique et la maladie de Pick.

Selon un mode de réalisation préféré, la présente invention concerne les composés de formule (I) susmentionnée pour leur utilisation pour le traitement de la maladie d'Alzheimer.

Selon un mode de réalisation, dans la formule (I) susmentionnée, au moins un des groupes R₁, R₂, R₃, R₄ et R₅ est différent de H.

De préférence, dans la formule (I) susmentionnée, l'un au moins des groupes R₁, R₂, R₃ et R₄ est différent de H.

Selon un mode de réalisation, dans la formule (I) susmentionnée, trois, voire quatre, parmi les groupes R₁, R₂, R₃ et R₄, est différent de H.

Selon un mode de réalisation préféré, dans la formule (I), R' est H.

Selon un mode de réalisation, dans la formule (I) telle que définie ci-dessus, R₅ est H. Parmi les composés préférés selon l'invention, on peut mentionner ceux répondant à la formule (I), dans laquelle R'=H et R₅=H.

Ainsi, une famille de composés préférés utilisés selon l'invention est constituée par des composés de formule (II) suivante :

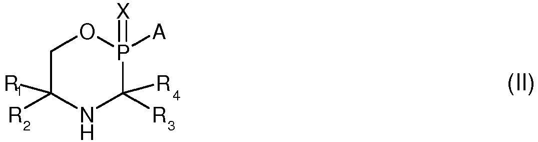

A, X, R₁, R₂, R₃ et R₄ étant tels que définis ci-dessus dans la formule (I).

Les composés de formule (II) correspondent à des composés de formule (I) dans laquelle R'=R₅=H.

Selon un mode de réalisation, dans la formule (I) telle que définie ci-dessus, R₁, R₂, R₃ et R₄, identiques ou différents, sont des groupes (C₁-C₆)alkyles.

De préférence, dans la formule (I) telle que définie ci-dessus, R₁, R₂, R₃ et R₄, identiques ou différents, sont des groupes (C₁-C₆)alkyles et R₅ est H.

Ainsi, une famille de composés préférés utilisés selon l'invention est constituée par des composés de formule (II-1) suivante :

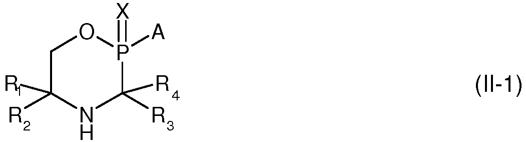

A et X étant tels que définis ci-dessus dans la formule (I), et R₁, R₂, R₃ et R₄ représentant des groupes (C₁-C₆)alkyles.

Les composés de formule (II-1) correspondent à des composés de formule (I) dans laquelle R'=R₅=H et R₁, R₂, R₃ et R₄ sont choisis parmi les groupes (C₁-C₆)alkyles.

Selon un mode de réalisation, les groupes R₁, R₂, R₃ et R₄ sont des groupes méthyles.

Parmi les composés préférés utilisés selon l'invention, on peut citer les composés de formule (I), dans laquelle X est O.

On peut également mentionner les composés de formules (II) et (II-1) susmentionnées dans laquelle X=O.

Selon un mode de réalisation préféré, les composés pour l'utilisation selon l'invention répondent à la formule (III) suivante :

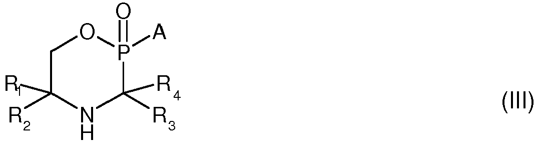

A, R₁, R₂, R₃ et R₄ étant tels que définis ci-dessus dans la formule (I).

De préférence, dans la formule (III) susmentionnée, R₁, R₂, R₃ et R₄ représentent des groupes (C₁-C₆)alkyles, et notamment des groupes méthyles.

De préférence, dans la formule (I) telle que définie ci-dessus, A est choisi parmi les groupes aryles et hétéroaryles tels que définis ci-dessus.

Une sous-famille de composés utilisés selon l'invention est donc constituée par des composés de formule (I) telle que définie ci-dessus, dans laquelle A est choisi dans le groupe constitué des groupes (C₆-C₁₀)aryles et des groupes hétéroaryles comprenant de 5 à 10 atomes.

Une autre sous-famille de composés utilisés selon l'invention est donc constituée par des composés de formule (II) telle que définie ci-dessus, dans laquelle A est choisi dans le groupe constitué des groupes (C₆-C₁₀)aryles et des groupes hétéroaryles comprenant de 5 à 10 atomes.

Une autre sous-famille de composés utilisés selon l'invention est donc constituée par des composés de formule (II-1) telle que définie ci-dessus, dans laquelle A est choisi dans le groupe constitué des groupes (C₆-C₁₀)aryles et des groupes hétéroaryles comprenant de 5 à 10 atomes.

Une autre sous-famille de composés utilisés selon l'invention est donc constituée par des composés de formule (III) telle que définie ci-dessus, dans laquelle A est choisi dans le groupe constitué des groupes (C₆-C₁₀)aryles et des groupes hétéroaryles comprenant de 5 à 10 atomes.

Selon un mode de réalisation, dans les formules (I), (II), (II-1) et (III) susmentionnées, A est un groupe aryle, et préférentiellement est un groupe phényle, éventuellement substitué.

Selon un mode de réalisation, A est un groupe phényle non substitué ou un groupe phényle substitué par au moins un substituant choisi dans le groupe constitué des atomes d'halogène, ORₐ, SRₐ, NO₂, NRₐR_{b}, N(Rₐ)COOR_{c}, R'ₐ et OR'ₐ ; Rₐ, R_{b}, R'ₐ et R_{c} étant tels que définis ci-dessus.

Selon un mode de réalisation, A est un groupe phényle substitué par au moins un substituant choisi dans le groupe constitué des atomes d'halogène, notamment Cl ou F, de NO₂, NRₐR_{b} et N(Rₐ)COOR_{c} ; Rₐ, R_{b} et R_{c} étant tels que définis ci-dessus.

Selon un mode de réalisation, dans les formules (I), (II), (II-1) et (III) susmentionnées, A est un groupe hétéroaryle tel que défini ci-dessus, éventuellement substitué.

De préférence, A est un groupe hétéroaryle comprenant 6 atomes dont au moins un atome d'azote. En particulier, A peut être choisi parmi les groupes pyridinyle et pyrimidinyle.

Selon un mode de réalisation, dans les formules (I), (II), (II-1) et (III) susmentionnées, A est choisi dans le groupe constitué des groupes 2-pyridinyle, 3-pyridinyle, 4-pyridinyle, 2-pyrimidinyle et 5-pyrimidinyle.

Parmi les composés utilisés selon l'invention, on peut citer en outre les composés de formule (I-1), (I-2) et (I-3) tels que définis ci-après.

L'invention concerne également des sous-familles de composés parmi les composés de formule (I). Ainsi, la présente invention concerne également un composé de formule (I-1) suivante : 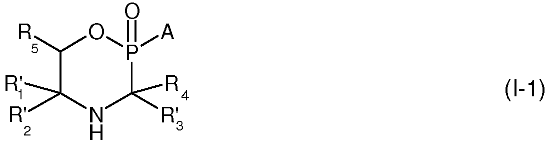 dans laquelle :
- R'₁, R'₂ et R'₃, identiques ou différents, sont des groupes (C₁-C₆)alkyles ou des groupes (C₆-C₁₀)aryles, R'₁, R'₂ et R'₃ étant de préférence des groupes méthyle,
- R₄ et R₅, identiques ou différents, sont choisis dans le groupe constitué de :
   - H,
   - des groupes (C₁-C₆)alkyles,
   - des groupes (C₆-C₁₀)aryles,
   R'₁ et R'₂ pouvant former ensemble, avec l'atome de carbone qui les porte, un cycle spiranique comprenant de 3 à 6 atomes de carbone, et/ou R'₃ et R₄ pouvant former ensemble, avec l'atome de carbone qui les porte, un cycle spiranique comprenant de 3 à 6 atomes de carbone,
- A est choisi dans le groupe constitué :
   - des groupes (C₆-C₁₀)aryles,
   - des groupes hétéroaryles comprenant de 5 à 10 atomes, et
   - des hétérocycloalkyles comprenant de 5 à 10 atomes,
      lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ ;
   Rₐ et R_{b}, identiques ou différents, étant H ou un groupe (C₁-C₆)alkyle ;
   R_{C} étant un radical -(C₁-C₆)alkylène-(C₆-C₁₀)aryle, notamment un radical -CH₂-(C₆-C₁₀)aryle ;
   R'ₐ étant choisi parmi les groupes CF₃, CHF₂ et CH₂F ;
   et, lorsque R'₁=R'₂=R'₃=Me, R₄=Me ou H et R₅=H, A est différent des groupes suivants :

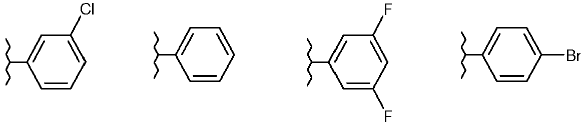

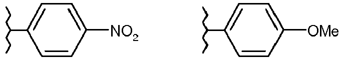

Les composés de formule (I-1) constituent une sous-famille de composés de formule (I) dans laquelle X=O et R'=H.

De préférence, dans la formule (I-1), R₅ est H.

Selon un mode de réalisation, dans la formule (I-1) telle que définie ci-dessus, R'₁, R'₂, R'₃ et R₄, identiques ou différents, sont des groupes (C₁-C₆)alkyles, de préférence méthyles.

De préférence, dans la formule (I-1) telle que définie ci-dessus, A est choisi parmi les groupes aryles et hétéroaryles tels que définis ci-dessus.

Une sous-famille de composés selon l'invention est donc constituée par des composés de formule (I-1) telle que définie ci-dessus, dans laquelle A est choisi dans le groupe constitué des groupes (C₆-C₁₀)aryles et des groupes hétéroaryles comprenant de 5 à 10 atomes.

Selon un mode de réalisation, dans la formule (I-1) susmentionnée, A est un groupe aryle, et préférentiellement est un groupe phényle, éventuellement substitué.

Selon un mode de réalisation, A est un groupe phényle non substitué ou un groupe phényle substitué par au moins un substituant choisi dans le groupe constitué des atomes d'halogène, ORₐ, SRₐ, NO₂, NRₐR_{b}, N(Rₐ)COOR_{c}, R'ₐ et OR'ₐ ; Rₐ, R_{b}, R'ₐ et R_{c} étant tels que définis ci-dessus.

Selon un mode de réalisation, A est un groupe phényle substitué par au moins un substituant choisi dans le groupe constitué des atomes d'halogène, notamment Cl ou F, de NO₂, NRₐR_{b} et N(Rₐ)COOR_{c} ; Rₐ, R_{b} et R_{c} étant tels que définis ci-dessus.

De préférence, dans la formule (I-1), A est choisi parmi les groupes suivants :

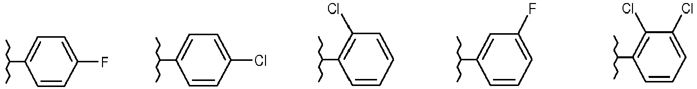

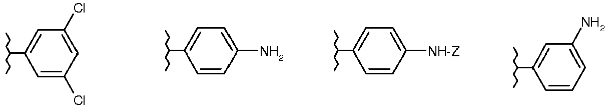

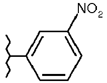

Selon un mode de réalisation, dans la formule (I-1) susmentionnée, A est un groupe hétéroaryle tel que défini ci-dessus, éventuellement substitué.

De préférence, dans la formule (I-1), A est un groupe hétéroaryle comprenant 6 atomes dont au moins un atome d'azote. En particulier, A peut être choisi parmi les groupes pyridinyle et pyrimidinyle.

Selon un mode de réalisation, dans la formule (I-1), A est choisi dans le groupe constitué des groupes 2-pyridinyle, 3-pyridinyle, 4-pyridinyle, 2-pyrimidinyle et 5-pyrimidinyle.

La présente invention concerne également un composé de formule (I-2) suivante : 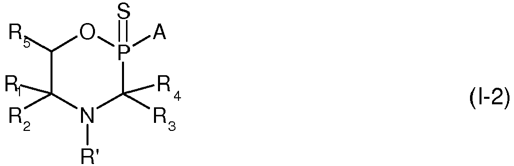 dans laquelle :
- A est choisi dans le groupe constitué :
   - des groupes (C₆-C₁₀)aryles,
   - des groupes hétéroaryles comprenant de 5 à 10 atomes, et
   - des hétérocycloalkyles comprenant de 5 à 10 atomes,
      lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ ;
      Rₐ et R_{b}, identiques ou différents, étant H ou un groupe (C₁-C₆)alkyle ;
      R_{C} étant un radical -(C₁-C₆)alkylène-(C₆-C₁₀)aryle, notamment un radical -CH₂-(C₆-C₁₀)aryle ;
      R'ₐ étant choisi parmi les groupes CF₃, CHF₂ et CH₂F ;
- R' est H ou un groupe (C₁-C₆)alkyle ;
- R₁, R₂, R₃, R₄ et R₅, identiques ou différents, sont choisis dans le groupe constitué de :
   - H,
   - des groupes (C₁-C₆)alkyles,
   - des groupes (C₆-C₁₀)aryles,
   R₁ et R₂ pouvant former ensemble, avec l'atome de carbone qui les porte, un cycle spiranique comprenant de 3 à 6 atomes de carbone, et/ou R₃ et R₄ pouvant former ensemble, avec l'atome de carbone qui les porte, un cycle spiranique comprenant de 3 à 6 atomes de carbone.

De préférence, dans la formule (I-2), R'=H. De préférence, dans la formule (I-2), R₅ est H et R₁, R₂, R₃, et R₄ sont des groupes alkyles, notamment méthyles.

De préférence, dans la formule (I-2), A est un groupe phényle, éventuellement substitué, notamment par au moins un atome d'halogène.

La présente invention concerne également un composé de formule (I-3) suivante : 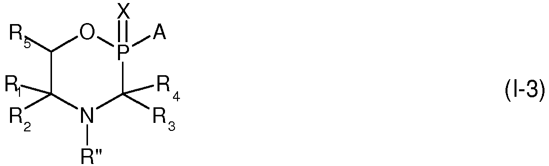 dans laquelle :
- X est O ou S ;
- A est choisi dans le groupe constitué :
   - des groupes (C₆-C₁₀)aryles,
   - des groupes hétéroaryles comprenant de 5 à 10 atomes, et
   - des hétérocycloalkyles comprenant de 5 à 10 atomes,
      lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ ;
      Rₐ et R_{b}, identiques ou différents, étant H ou un groupe (C₁-C₆)alkyle ;
      R_{C} étant un radical -(C₁-C₆)alkylène-(C₆-C₁₀)aryle, notamment un radical -CH₂-(C₆-C₁₀)aryle ;
      R'ₐ étant choisi parmi les groupes CF₃, CHF₂ et CH₂F ;
- R" est un groupe (C₁-C₆)alkyle ;
- R₁, R₂, R₃, R₄ et R₅, identiques ou différents, sont choisis dans le groupe constitué de :
   - H,
   - des groupes (C₁-C₆)alkyles,
   - des groupes (C₆-C₁₀)aryles,
   R₁ et R₂ pouvant former ensemble, avec l'atome de carbone qui les porte, un cycle spiranique comprenant de 3 à 6 atomes de carbone, et/ou
   R₃ et R₄ pouvant former ensemble, avec l'atome de carbone qui les porte, un cycle spiranique comprenant de 3 à 6 atomes de carbone.

De préférence, dans la formule (I-3), X=O. De préférence, dans la formule (I-3), R₅ est H et R₁, R₂, R₃, et R₄ sont des groupes alkyles, notamment méthyles.

De préférence, dans la formule (I-3), A est un groupe phényle, éventuellement substitué, notamment par au moins un atome d'halogène.

Parmi les composés de formule (I), (I-1), (I-2) ou (I-3), on peut notamment mentionner les composés suivants :

| | | |
|---|---|---|
| 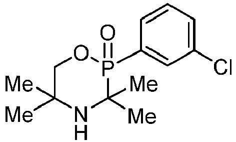 | 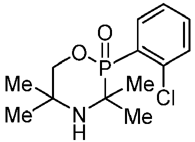 | 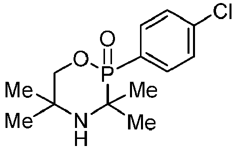 |
| **3b** | **3a** | **3c** |
| 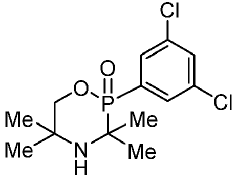 | 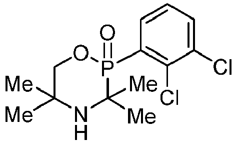 | 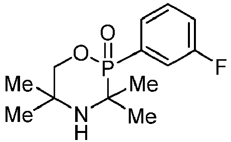 |
| **3d** | **3e** | **3f** |
| 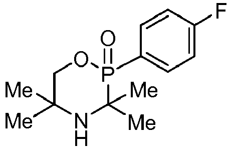 | 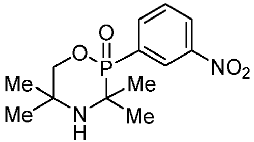 | 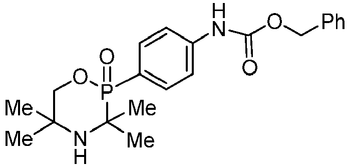 |
| **3g** | **3h** | **3i** |
| 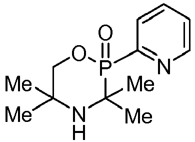 | 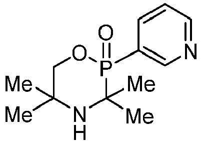 | 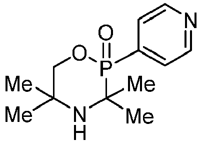 |
| **3j** | **3k** | **3l** |
| 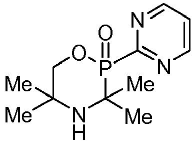 | 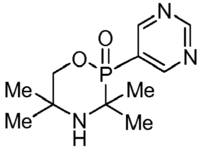 | 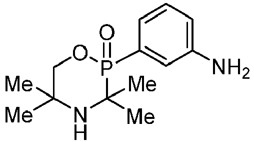 |
| **3m** | **3n** | **6** |
| 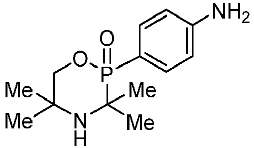 | 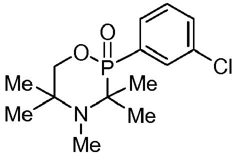 | 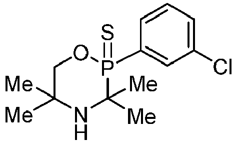 |
| **7** | **8** | **9** |

Les composés selon l'invention présentent une activité neuroprotective. Les composés selon l'invention peuvent donc être utilisés pour la préparation de médicaments.

Ainsi, l'invention concerne également des médicaments qui comprennent un composé de formule (I-1), (I-2) ou (I-3), ou un sel d'addition de ce dernier à un acide pharmaceutiquement acceptable.

Ces médicaments trouvent leur emploi en thérapeutique, notamment dans le traitement de maladies neurodégénératives.

La présente invention concerne également une composition pharmaceutique, comprenant au moins un composé de formule (I-1), (I-2) ou (I-3) telles que définies ci-dessus, en association avec au moins un véhicule ou excipient pharmaceutiquement acceptable.

La présente invention concerne donc également des compositions pharmaceutiques comprenant, en tant que principe actif, un composé selon l'invention, à savoir un composé répondant à l'une des formules (I-1), (I-2) ou (I-3). Ces compositions pharmaceutiques contiennent une dose efficace d'au moins un composé selon l'invention, ou un sel pharmaceutiquement acceptable, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I-1), (I-2) ou (I-3) ci-dessus, ou son sel, peut être administré sous forme unitaire d'administration, en mélange avec des excipients pharmaceutiques classiques, aux animaux et aux êtres humains pour le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

La présente invention concerne un composé de formule (I-1), (I-2) ou (I-3) telle que définie ci-dessus, ou un sel d'addition de ce composé à un acide pharmaceutiquement acceptable, pour son utilisation à titre de médicament.

La présente invention, selon un autre de ses aspects, concerne également une méthode de traitement des maladies neurodégénératives ci-dessus indiquées qui comprend l'administration, à un patient, d'une dose efficace d'un composé selon l'invention, ou un de ses sels pharmaceutiquement acceptables.

### FIGURES

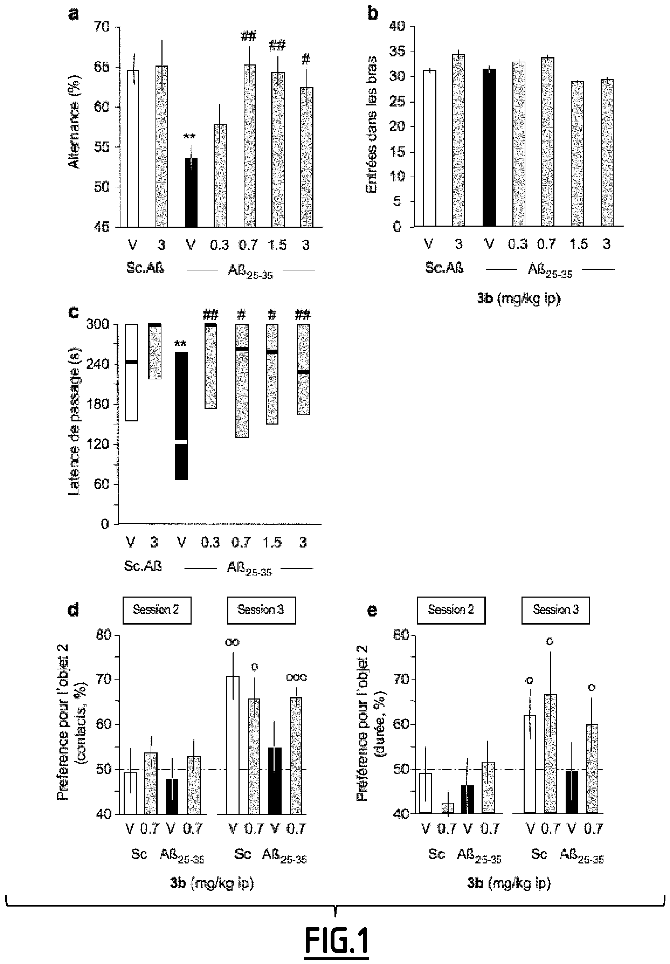

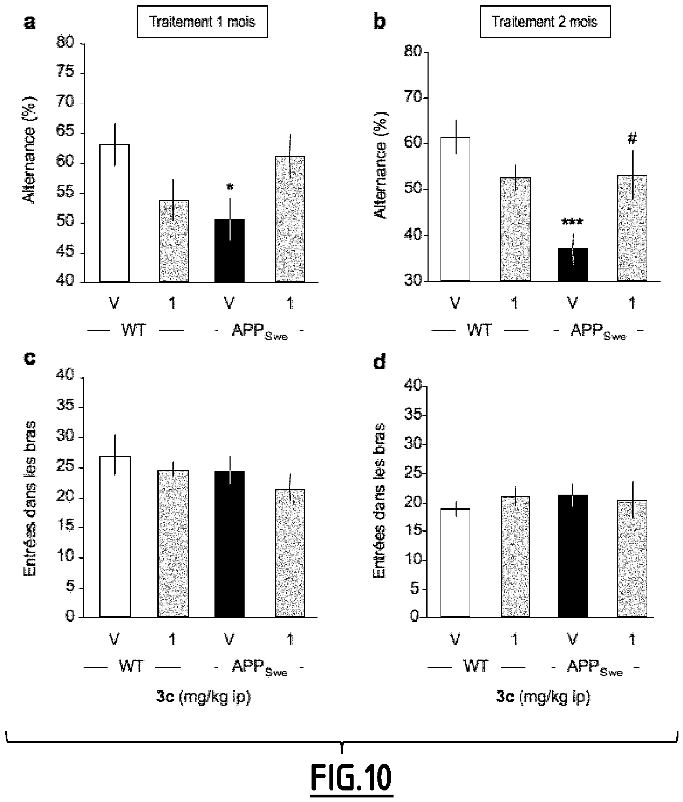

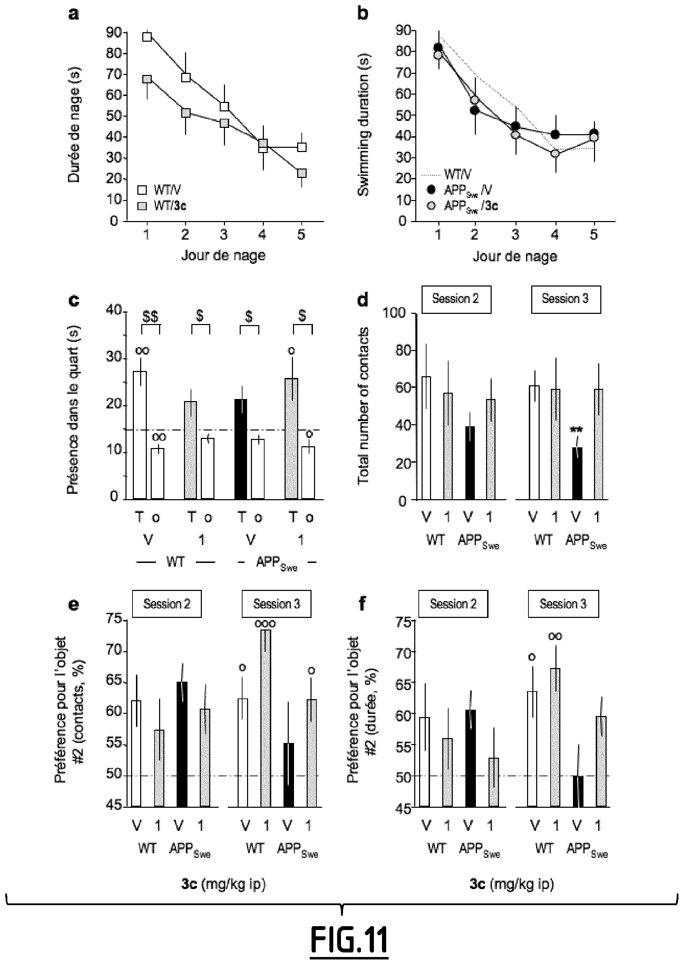

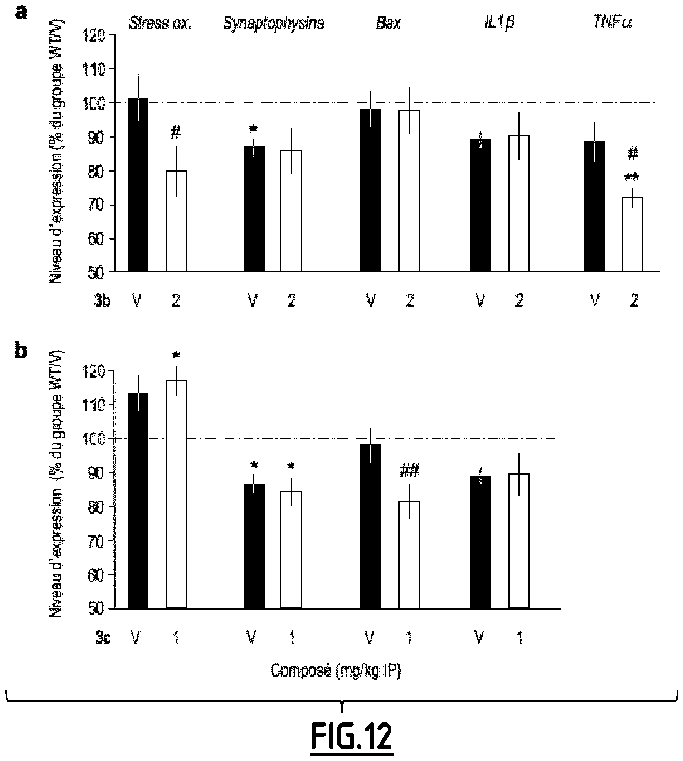

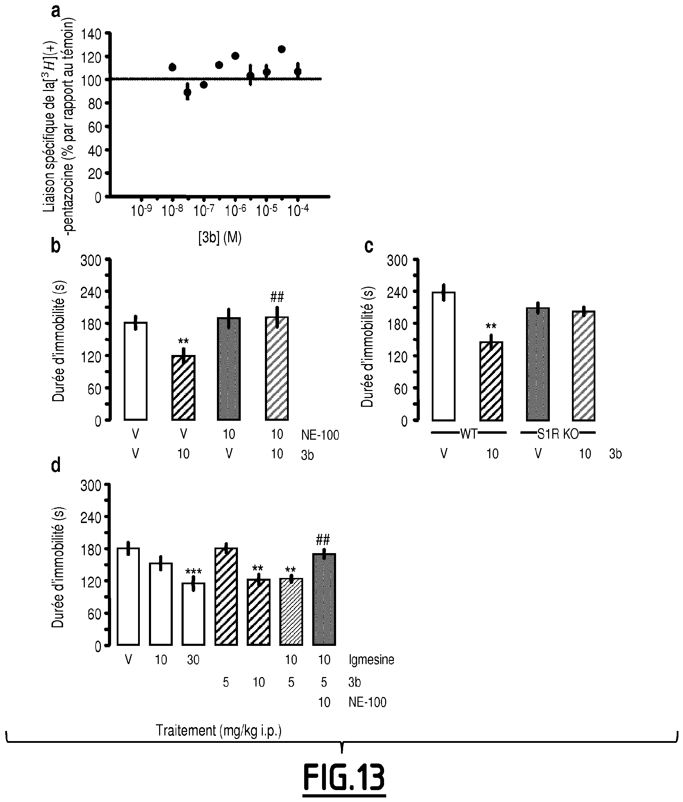

**Figure 1** **:** Effets comportementaux induits par le composé 3b chez les souris traitées au Aβ₂₅₋₃₅ : (a, b) alternance spontanée dans le labyrinthe en Y, (c) réponse d'évitement passif, et (d, e) test de reconnaissance d'objet. Les animaux ont été traités avec le composé 3b (0.3-3 mg/kg IP) 20 min avant le peptide Aβ₂₅₋₃₅ ou Sc.Aβ (9 nmol ICV). Après 7 jours, les souris ont été testées au labyrinthe en Y : (a) performance d'alternance spontanée et (b) nombre total d'entrées dans les bras ; puis au test d'évitement passif : (c) latence de passage ; puis au test de reconnaissance d'objet : analyse de la préférence pour l'objet 2 (nouveau lors de la session 3) calculée à partir (d) du nombre de contacts avec les objets et (e) de la durée de contact. ANOVA: F_{(6,167)} = 4.58, p < 0.001 en (a); F_{(6,167)} = 2.65, p < 0.05 in (b); H = 20.5, p < 0.01 en (c), n = 18-30 par groupe. ** p < 0.01 par rapport au groupe (Sc.Aβ+V) ; # p < 0.05, ## p < 0.01 par rapport au groupe (Aβ₂₅₋₃₅+V) ; test de Dunnett en (a) et test de Dunn en (c). ° p < 0.05, °° p < 0.01, °°° p < 0.001 par rapport au niveau de chance (50 s); test t, n = 4-13 en (d, e).

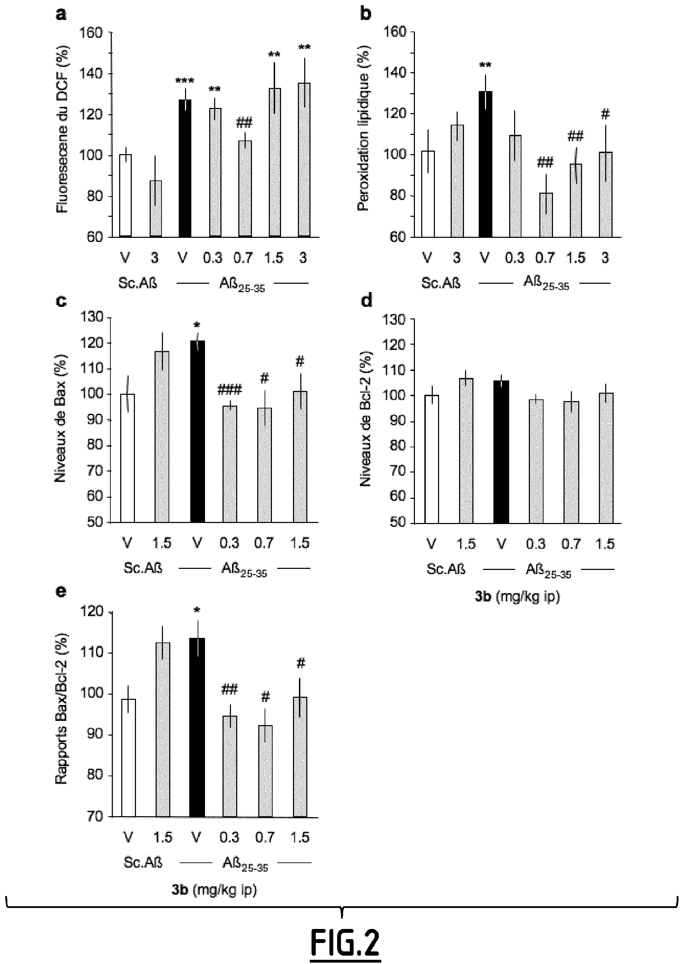

**Figure 2** **:** Effet neuroprotecteur du composé 3b chez les souris traitées au Aβ₂₅₋₃₅ : (a) Niveau des espèces réactives de l'oxygène dans l'hippocampe, (b) Niveau de peroxydation lipidique dans le cortex frontal, (c) Niveau de protéine Bax dans l'hippocampe, (d) Niveau de Bcl-2 et (e) rapport Bax/Bcl-2. Les souris ont été traitées avec le composé 3b (0.3-3 mg/kg IP) 20 min avant le peptide Aβ₂₅₋₃₅ ou Sc.Aβ (9 nmol ICV) et sacrifiées après 9 jours. Les valeurs sont exprimées en % du groupe contrôle (Sc.Aβ+V). ANOVA: F_{(6,47)} = 2.60, p < 0.05, n = 6-12 par groupe en (a); F_{(6,62)} = 5.03, p < 0.001, n = 7-13 en (b); F_{(5,36)} = 3.36, p < 0.05 en (c); F < 1 en (d); F_{(5,36)} = 3.72, p < 0.01, n = 5-6 en (e). * p < 0.05, ** p < 0.01, *** p < 0.001 par rapport au groupe (Sc.Aβ+V) ; # p < 0.05, ## p < 0.01, ### p < 0.001 par rapport au groupe (Aβ₂₅₋₃₅+V) ; test de Dunnett.

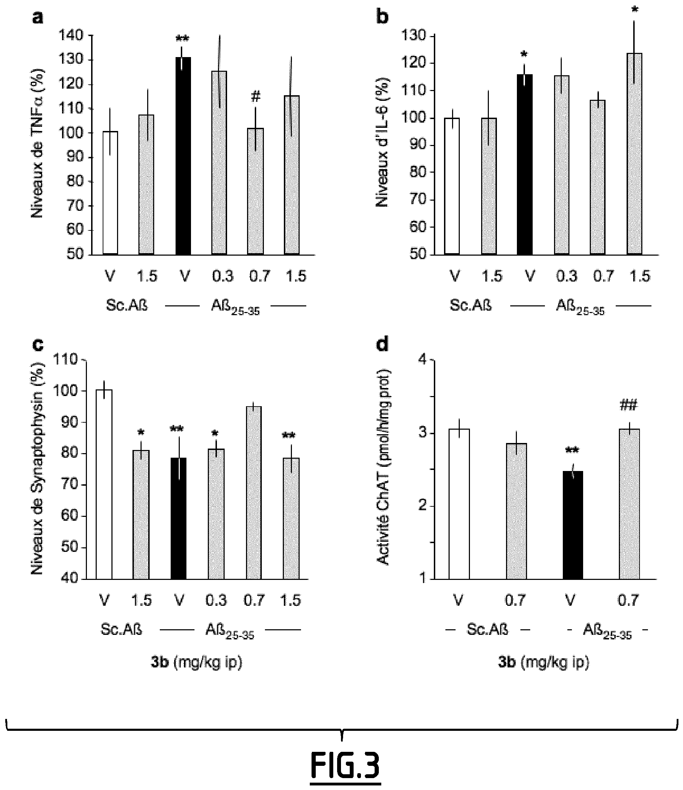

**Figure 3** **:** Effet neuroprotecteur du composé 3b dans l'hippocampe de souris traitées au Aβ₂₅₋₃₅ : (a) taux de TNFα, (b) taux d'IL1β, (c) niveaux de synaptophysine (c) et activité ChAT (d). Les souris ont été traitées avec le composé 3b (0.3-3 mg/kg IP) 20 min avant le peptide Aβ₂₅₋₃₅ ou Sc.Aβ (9 nmol ICV) et sacrifiées après 9 jours. Les valeurs sont exprimées en % du groupe contrôle (Sc.Aβ+V). ANOVA: F_{(5,56)} = 1.09, p > 0.05, n = 7-11 par groupe en (a); F_{(5,67)} = 3.82, p < 0.01, n = 5-6 en (b); F_{(5,30)} = 4.55, p < 0.01 en (c); F_{(3,23)} = 4.97, p < 0.01 en (d). * p < 0.05, ** p < 0.01 par rapport au groupe (Sc.Aβ+V) ; # p < 0.05, ## p < 0.01 par rapport au groupe (Aβ₂₅₋₃₅+V) ; test de Dunnett.

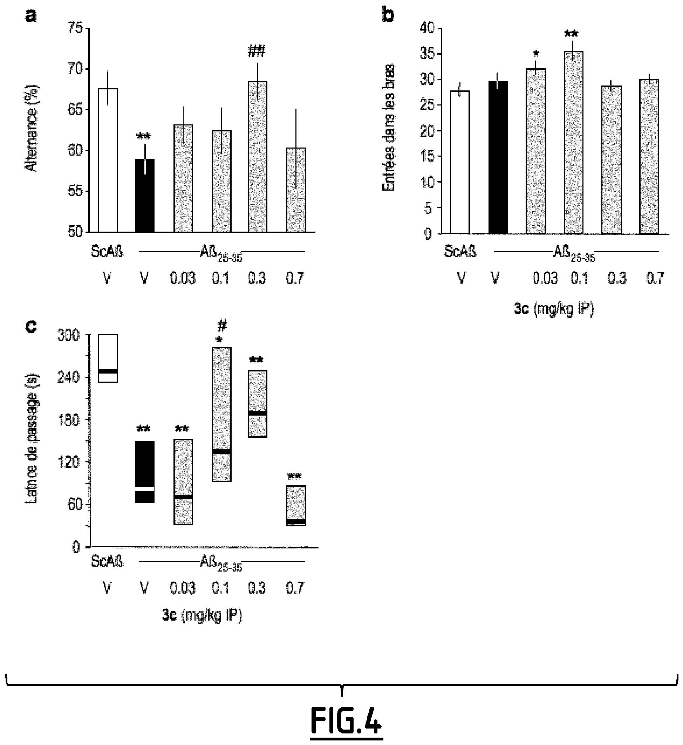

**Figure 4** **:** Effets du composé 3c chez les souris traitées avec le peptide Aβ₂₅₋₃₅ : (a, b) alternance spontanée dans le labyrinthe en Y, et (c) évitement passif. Les souris ont été traitées avec le composé 3c (0.03-0.7 mg/kg IP) 20 min avant le peptide Aβ₂₅₋₃₅ ou Sc.Aβ (9 nmol ICV). Après 7 jours, les souris ont été testées au labyrinthe en Y : (a) performance d'alternance spontanée et (b) nombre total d'entrées dans les bras ; puis au test d'évitement passif : (c) latence de passage. ANOVA : F_{(5,67)} = 2.54, p < 0.05 en (a); F_{(5,67)} = 3.80, p < 0.01 en (b); H = 21.4, p < 0.001 en (c), n = 4-14 par groupe. * p < 0.05, ** p < 0.01 par rapport au groupe (Sc.Aβ+V) ; # p < 0.05, ## p < 0.01 par rapport au groupe (Aβ₂₅₋₃₅+V) ; test de Dunnett en (a,b) et de Dunn en (c).

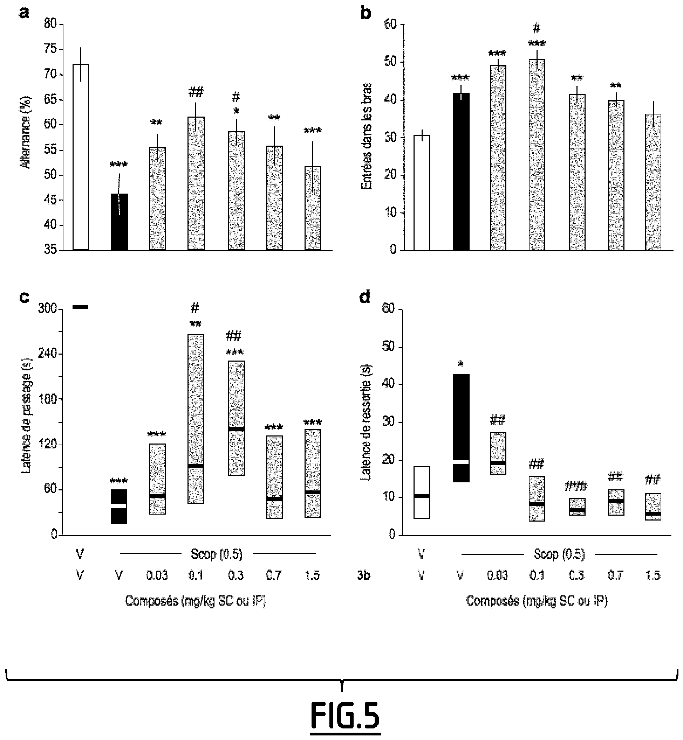

**Figure 5** **:** Effet anti-amnésiant du composé 3b chez les souris traitées à la scopolamine : (a, b) alternance spontanée dans le labyrinthe en Y, et (c,d) évitement passif. Les souris ont reçu le composé 3b (0.03-1.5 mg/kg IP) 10 min avant la scopolamine (0.5 mg/kg SC), 20 min avant la session de labyrinthe en Y ou l'entrainement à l'évitement passif. Test du labyrinthe en Y : (a) alternance spontanée et (b) nombre total d'entrées dans les bras ; rétention à l'évitement passif : (c) latence de passage et (d) latence de ressortie. ANOVA: F(6,94) = 5.32, p < 0.0001 en (a); F(6,94) = 10.6, p < 0.0001 en (b) ; H = 31.4, p < 0.0001 en (c); H = 24.3, p < 0.001 en (d); n = 12-19 par groupe. * p < 0.05, ** p < 0.01, *** p < 0.001 par rapport au groupe (Sc.Aβ+V) ; # p < 0.05, ## p < 0.01, ### p < 0.001 par rapport au groupe (Aβ25-35+V) ; test de Dunnett en (a, b) et de Dunn en (c, d).

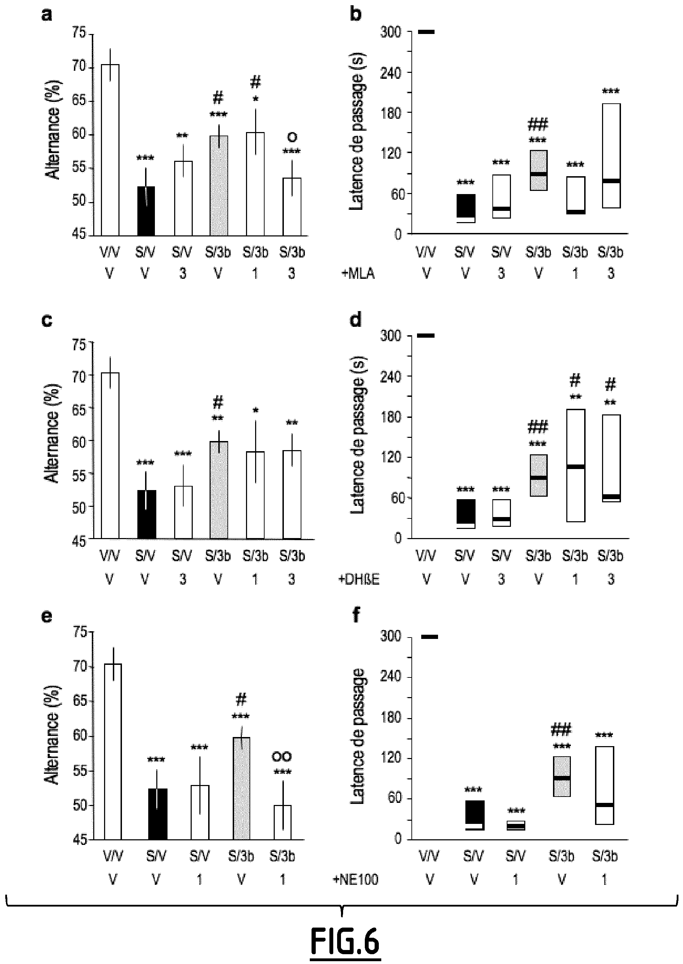

**Figure 6** **:** Analyse de l'antagonisme des effets anti-amnésiants du composé 3b chez les souris traitées à la scopolamine : (a, c, e) alternance spontanée dans le labyrinthe en Y, et (b, d, f) latence de passage lors de la session de rappel à l'évitement passif. Les souris ont été traitées avec la méthyllycaconitine (MLA, 1 ou 3 mg/kg IP), la dihydro-β-érythroïdine (DHβE, 1 ou 3 mg/kg IP), ou le NE100 (1 mg/kg IP), simultanément avec le composé 3b (0.1 mg/kg IP) 10 min avant la scopolamine (S, 0.5 mg/kg SC), 20 min la session comportementale. ANOVA: F_{(5,87)} = 7.19, p < 0.0001 en (a) ; H = 31.3, p < 0.0001 en (b) ; F_{(5,82)} = 5.82, p < 0.0001 en (c); H = 28.8, p < 0.0001 en (d) ; F_{(4,77)} = 10.0, p < 0.0001 en (e); H = 30.5, p < 0.0001 en (f); n = 12-17 par groupe. * p < 0.05, ** p < 0.01, *** p < 0.001 par rapport au groupe (Sc.Aβ+V) ; # p < 0.05, ## p < 0.01, ### p < 0.001 par rapport au groupe (Aβ₂₅₋₃₅+V) ; test de Dunnett en (a, b) et de Dunn en (c, d).

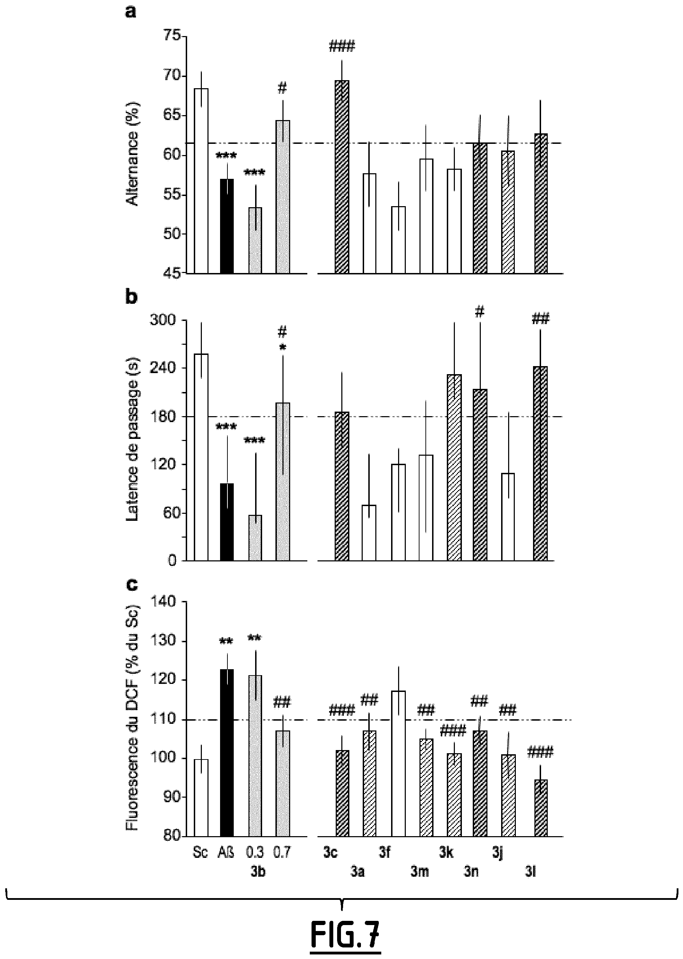

**Figure 7** **:** Criblage des phosphinolactones comme neuroprotecteurs chez les souris traitées avec le peptide Aβ₂₅₋₃₅ : (a) alternance spontanée ; (b) latence de passage à l'évitement passif; (c) Niveaux d'espèces réactives de l'oxygène dans l'hippocampe. Les souris ont reçu le composé 3b (0.3, 0.7 mg/kg IP) ou une dose de chaque composé (0.3 mg/kg IP) 20 min avant l'Aβ₂₅₋₃₅ ou le peptide contrôle Sc.Aβ (9 nmol ICV). Les animaux ont été testés dans le labyrinthe en Y au jour 7 après les injections ICV, à l'évitement passif aux jours 8-9 et sacrifiés. Dans chaque graphe en (a-c), le niveau 50% de protection par rapport au déficit induit par Aβ₂₅₋₃₅ est tracé en pointillé. Noter qu'en (a), la colonne représente la médiane et les barres d'erreurs les écarts 25% - 75% (= écarts asymétriques). n = 12 en (a, b) et 5-6 en (c). ANOVA sur les groupes 1-4: F_{(3,63)} = 7.95, p < 0.001 en (b); H = 24.1, p < 0.0001 en (c) ; F_{(3,44)} = 6.33, p < 0.01 en (d). * p < 0.05, ** p < 0.01, *** p < 0.001 par rapport au groupe (Sc.Aβ+V) ; # p < 0.05, ## p < 0.01, ### p < 0.001 par rapport au groupe (Aβ₂₅₋₃₅+V) ; test de Dunnett en (a, c) et de Dunn en (b). Les valeurs des groupes 5-12 (composés criblés) ont été analysées par un test t en (a, c) ou de Mann-Whitney en (b).

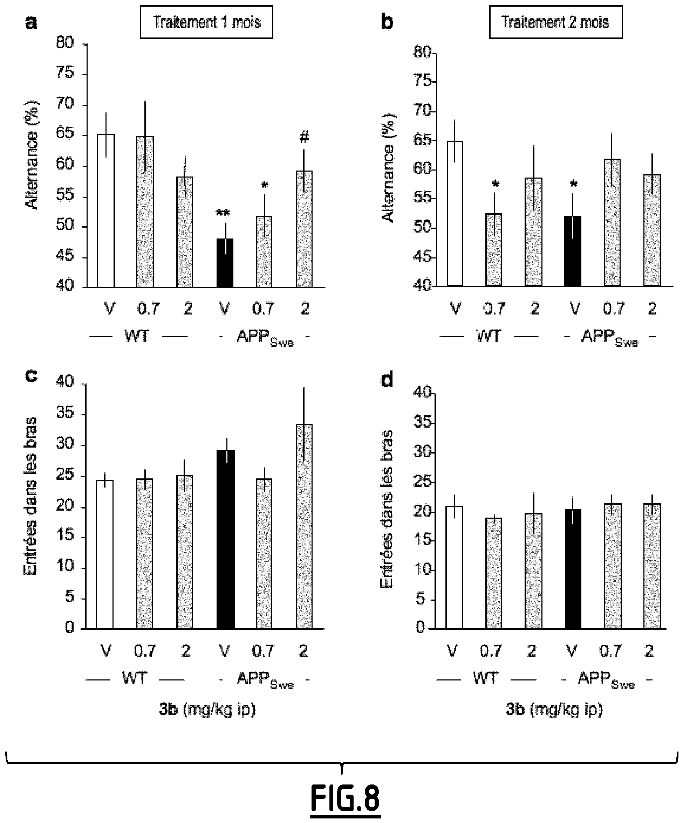

**Figure 8** : Alternance spontanée (a, b) et nombre total d'entrées dans les bras (c, d) de souris APP_{Swe} administrées avec le composé **3b**, dans le labyrinthe en Y, après 1 mois (a, c) et 2 mois (b, d) de traitement. Les animaux ont reçu le composé **3b** (0.7 ou 2 mg/kg IP) 3 fois par semaine pendant 1 ou 2 mois. N = 6-15 par groupe. ANOVA: F_{(5,49)} = 3.84, p < 0.01 en (a); F_{(5,54)} = 1.54, p > 0.05 en (b); F < 1 en (c) et (d). * p < 0.05, ** p < 0.01 par rapport au groupe (WT+V) ; # p < 0.05 par rapport au groupe (APP_{Swe}+V) ; test de Dunnett en (a, b).

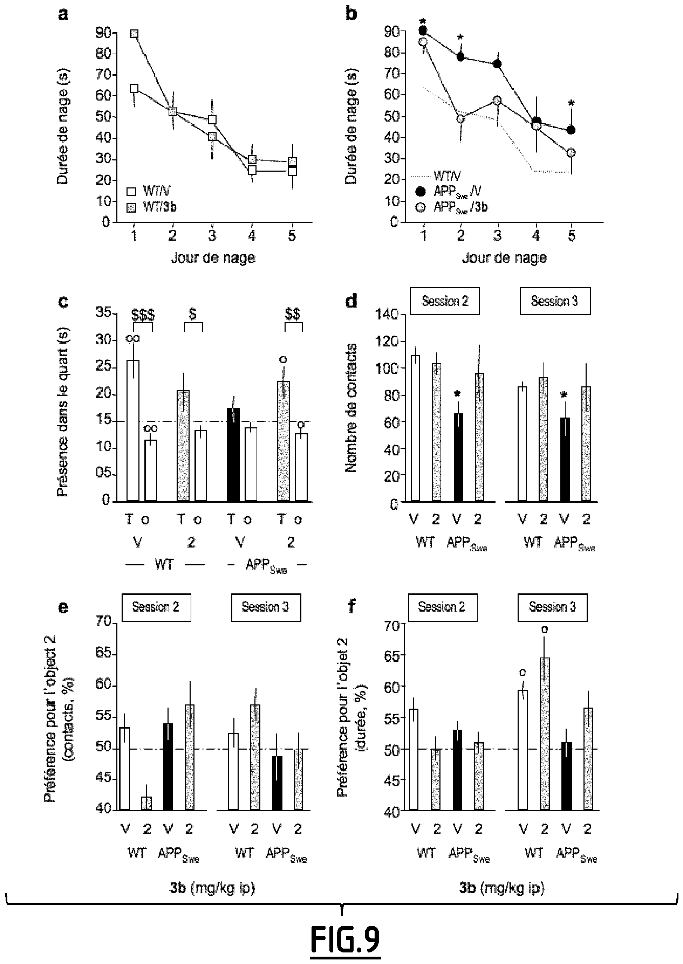

**Figure 9** **:** Apprentissage spatial en piscine (a-c) et reconnaissance d'objet (d-f) pour les souris APP_{Swe} après 2 mois de traitement avec le composé **3b.** Piscine : profils d'acquisition des souris WT traitées avec V et 3b (2 mg/kg) (a) ou des souris APP_{Swe} (b). (c) Présence dans le quart d'entrainement (T) et moyenne dans les autres quarts (o) pendant le test d'essai fait 48 h après la dernière session d'entrainement. ANOVA non-paramétrique en mesures répétés de Friedman : Fr = 21.7, p < 0.001 pour le groupe WT/V, Fr = 21.8, p < 0.001 pour le groupe WT/3b en (a); Fr = 13.0, p < 0.05 pour le groupe APP_{Swe}/V, Fr = 12.2, p < 0.05 pour le groupe APP_{Swe}/3b en (b). Reconnaissance d'objet : Nombre de contacts avec les objets durant les sessions 2 et 3 (d) et préférences pour l'objet en position 2 calculée en contacts (e) ou en durée de contact (f). ° p < 0.05, °° p < 0.01 par rapport au niveau de hasard : 15 s en (c), 50% en (e,f), test t à un échantillon. ^{$} p < 0.05, ^{$$} p < 0.01, ^{$$$} p < 0.001 par rapport à la moyenne des autres quarts (o) en (c). * p < 0.05 par rapport au groupe WT/V en (d).
**Figure 10** : Alternance spontanée (a, b) et nombre d'entrées dans les bras (c, d) pour les souris APP_{Swe} traitées avec le composé **3c**, dans le labyrinthe en Y, après 1 mois (a, c) ou 2 mois (b, d) de traitement. Les animaux ont reçu du composé **3c** (1 mg/kg IP) 3 fois par semaine pendant 1 ou 2 mois. N = 10-13 par groupe. ANOVA: F_{(3,45)} = 3.61, p < 0.05 en (a); F_{(3,38)} = 6.33, p < 0.01 en (b); F < 1 en (c) et (d). * p < 0.05, *** p < 0.001 par rapport au groupe WT/V; # p < 0.05 par rapport au groupe APP_{Swe}/V; test de Dunnett en (a, b).
**Figure 11** : Apprentissage spatial en piscine (a-c) et reconnaissance d'objet (d-f) pour les souris APP_{Swe} après 2 mois de traitement avec le composé **3c.** Piscine : profils d'acquisition des souris WT traitées avec V et **3c** (2 mg/kg) (a) ou des souris APP_{Swe} (b). (c) Présence dans le quart d'entraitement (T) et moyenne dans les autres quarts (o) pendant le test d'essai fait 48 h après la dernière session d'entrainement. ANOVA non-paramétrique en mesures répétés de Friedman : Fr = 20.7, p < 0.001 pour le groupe WT/V, Fr = 17.5, p < 0.001 pour le groupe WT/3c en (a); Fr = 11.4, p < 0.05 pour le groupe APP_{Swe}/V, Fr = 13.6, p < 0.01 pour le groupe APP_{Swe}/3b en (b). Reconnaissance d'objet : Nombre de contacts avec les objets durant les sessions 2 et 3 (d) et préférences pour l'objet en position 2 calculée en contacts (e) ou en durée de contact (f). ° p < 0.05, °° p < 0.01, °°° p < 0.001 par rapport au niveau de hasard : 15 s en (c), 50% en (e,f), test t à un échantillon. ^{$} p < 0.05, ^{$$} p < 0.011 par rapport à la moyenne des autres quarts (o) en (c). * p < 0.05 par rapport au groupe WT/V en (d).
**Figure 12** : Analyses biochimiques de l'effet des traitements avec le composé **3b** (a) ou **3c** (b) dans l'hippocampe des souris APP_{Swe}. Le stress oxydant a été mesuré par le niveau de peroxydation des lipides membranaires en (a), ou la fluorescence du DCF en (b). Les niveaux d'expression de la synaptophysine, de Bax, de l'IL1β et du TNFα ont été mesurés par des kits Elisa commerciaux. Les valeurs sont présentées en % du groupe WT/V contrôle. N = 6-8 par groupe. * p < 0.05, ** p < 0.01 par rapport au groupe WT/V; # p < 0.05, ## p < 0.01 par rapport au groupe APP_{Swe}/V; test t de Student.
**Figure 13** **:** Tests du composé 3b comme modulateur positif de la protéine sigma-1. (a) Le composé 3b ne modifie pas la fixation de la [³H](+)-pentazocine sur le récepteur sigma-1, dans des préparations de membranes de cerveau antérieur de cobaye, dans une gamme de concentration de 10 nM à 100 µM. (b) L'effet antidépresseur du composé 3b dans le test de nage forcée chez la souris est bloqué par l'antagoniste sélectif NE-100. Les souris Swiss mâles de 7 semaines d'âge (n = 10-12 par groupe) ont été forcées de nager pendant 15 min le jour 1 et 6 min le jour 2. Elles ont reçu du NE-100 (10 mg/kg ip), 10 min avant le composé 3b (10 mg/kg ip) et ce, 20 min avant la session du jour 2. L'immobilité a été mesurée durant les 5 dernières minutes de la session. La solution véhicule est l'eau distillée. F_{(3,42)} = 4.45, p < 0.01; ** p< 0.01 vs. groupe V/V, ## p < 0.01 vs. groupe V/3b, test de Dunnett. (c) Absence d'effet antidépresseur du composé 3b chez les souris KO pour la protéine sigma-1 (S1R KO). Les souris de souche sauvage (Wt, n = 14-15) et les souris S1R KO (n = 9-10) ont été forcées de nager pendant 15 min le jour 1 et 6 min le jour 2. Elles ont reçu le composé 3b (10 mg/kg ip) 20 min avant la session du jour 2. L'immobilité a été mesurée durant les 5 dernières minutes de la session. La solution véhicule est l'eau distillée. F_{(3,49)} = 6.64, p < 0.001; ** p< 0.01 vs. groupe Wt/V, test de Dunnett. (d) Potentiation des effets d'un agoniste sigma-1 par le composé 3b dans le test de nage forcée. Les souris Swiss mâles de 7 semaines d'âge (n = 8-23 par groupe) ont été forcées de nager pendant 15 min le jour 1 et 6 min le jour 2. Elles ont reçu de l'igmésine (10, 30 mg/kg ip) et/ou le composé 3b (10 mg/kg ip) 20 min avant la session du jour 2. Le NE-100 (10 mg/kg ip) a été injecté 10 min avant l'igmésine et le composé 3b. L'immobilité a été mesurée durant les 5 dernières minutes de la session. La solution véhicule est l'eau distillée. F_{(6,90)} = 5.59, p < 0.0001; , *** p< 0.001 vs. groupe V, ## p < 0.01 vs. groupe V/Igmesine (10)+3B (5), test de Dunnett.

Les exemples suivants décrivent la préparation de certains composés conformes à l'invention. Ces exemples ne sont pas limitatifs et ne font qu'illustrer la présente invention.

### EXEMPLES

### PRÉPARATION DE COMPOSÉS SELON L'INVENTION

### Généralités sur le procédé de préparation des composés de l'invention

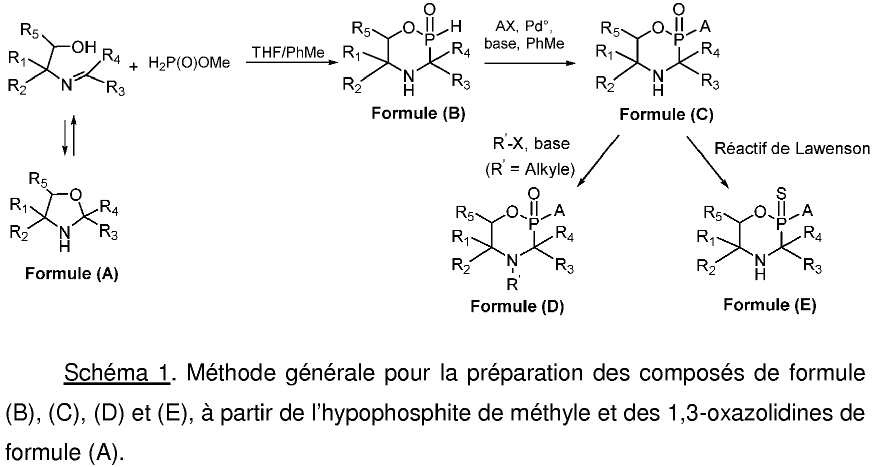

D'une manière classique, les structures ayant la formule (C) peuvent être préparées par arylation pallado-catalysée de précurseurs H-1,4,2-oxazaphosphinanes de formule (B). Les composés de formule (B) sont accessibles par une réaction d'addition cyclisation de l'hypophosphite de méthyle [Cristau, H.-J.; Coulombeau, A.; Genevois-Borella, A.; Pirat, J.-L. Tetrahedron Lett. 2001, 42, 4491-4494] avec la forme iminoalcools des 1,3-oxazolidines de formule (A) [Volle, J.-N., Filippini, D., Krawczy, B., Kaloyanov, N., Van der Lee, A., Maurice, T., Pirat, J.-L., Virieux, D. Org. Biomol. Chem. 2010, 8, 1438-1444 ; Cristau H.-J., J. Monbrun, Monique Tillard, J.-L. Pirat, Tetrahedron Lett. 2003, 3183-3186 ; Pirat J.-L., Monbrun J., Virieux D., Cristau H.-J. Tetrahedron 2005, 7029-7036 ; Volle, J.-N., Virieux, D., Starck, M., Monbrun, J., Clarion, L., Pirat J.-L. Tetrahedron Asymmetry 2006, 1402-1408 ; et Volle, J.-N., Kaloyanov, N., Saada, M. C., Virieux, D., Pirat, J.-L. Tetrahedron lett. 2007, 48, 4695-4697].

Les dérivés de type N-alkyle de formule (D) (R' = alkyle) peuvent être accessibles par réaction de N-alkylation des dérivés amines secondaires correspondants (C). Quant aux dérivés thiono (P=S, formule E), ils peuvent être obtenus par traitement des dérivés oxo (P=O, C) avec le réactif de Lawesson.

Une voie alternative peut être utilisée pour l'accès aux composés de formule (C). Elle consiste à une condensation directe en présence d'une base, d'un aryl-H-phosphinate d'alkyle sur une oxazolidine adéquate de formule (A). Cette stratégie a été précédemment décrite dans le journal "Organic and Biomolecular Chemistry" [Volle, J.-N., Filippini, D., Krawczy, B., Kaloyanov, N., Van der Lee, A., Maurice, T., Pirat, J.-L., Virieux, D. Org. Biomol. Chem. 2010, 8, 1438-1444].

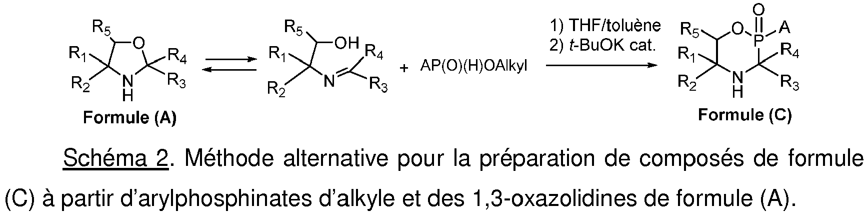

Par exemple, les dérivés hétérocycliques **3** avec R¹, R², R³, R⁴ et R⁵ = H ; R' = H et A = aryle ou hétéroaryle, ont pu être obtenus en utilisant une séquence réactionnelle de deux étapes. La première étape a consisté en une condensation-cyclisation de l'hypophosphite de méthyle sur le 2,2,4,4-tétraméthyl-1,3-oxazolidine, pour produire le H-oxazaphosphinane **5.** Ce dérivé à liaison P-H a été alors transformé par arylation pallado-catalysée en dérivés 2-aryl-oxazaphosphinanes **3** (Schéma 3). Des transformations subséquentes en présence d'hydrogène des composés **3** porteurs de motifs aryliques, tel que Ar = *m*-NO₂-C₆H₄ et *p*-CBzNH-C₆H₄, ont fourni les produits **6** et **7** porteur d'un groupement NH₂. A partir du composé **3** (Ar = *m*-ClC₆H₄), une réaction de méthylation avec l'iodure de méthyle a donné le dérivé *N*-méthyl oxazaphosphinane **8**, et l'utilisation du réactif de Lawesson, a fourni le composé thiono **9** (Schéma 3).

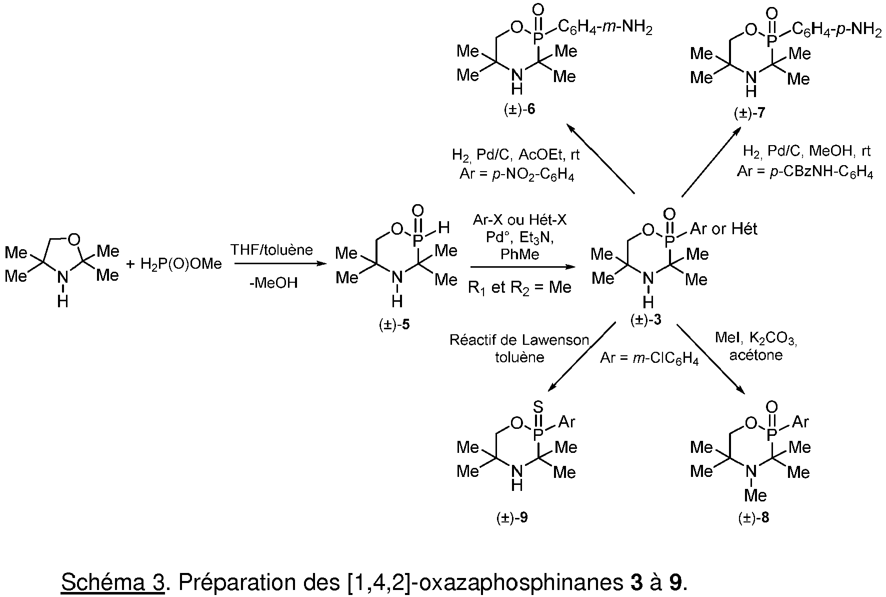

### Matériels et méthodes

Les solvants et les produits chimiques utilisés dans les réactions proviennent des fournisseurs tels que CARLO ERBA, Sigma-Aldrich, Alfa Aesar, Acros, ...

Pour réaliser les réactions, les solvants ont été séchés, distillés et stockés sous atmosphère d'azote. Toutes les réactions utilisant des réactifs sensibles à l'air ou à l'humidité ont été menées sous atmosphère d'azote avec une verrerie séchée par chauffage sous vide. Les points de fusion n'ont pas été corrigés. Les spectres de Résonance Magnétique Nucléaire (RMN) ont été réalisés sur un spectromètre de la marque Bruker (400 MHz), opérant à la fréquence de 400,1 MHz pour le ¹H, 100,6 MHz pour le ¹³C, 162,0 MHz pour le ³¹P et 376,5 MHz pour le ¹⁹F. Les déplacements chimiques δ de chaque noyau sont exprimés en ppm, les constantes de couplage en Hz. Pour les spectres du ¹H, le signal du chloroforme a été calibré à 7,26 ppm et 2,50 ppm dans le cas du diméthylsulfoxyde. Pour les spectres du ¹³C, le signal du carbone du chloroforme deutéré a été calibré à 77,16 ppm et 39,52 ppm pour le dimethylsulfoxide-d₆. Toutes les expériences de RMN réalisées sur le noyau du phosphore ou le noyau du fluor ont été indiquées sans couplage avec l'hydrogène. Le spectromètre de masse SYNAPT G2-S de la marque Water a été utilisé pour l'obtention des Spectres de Masse en Haute Résolution (SM-HR).

### Exemple 1 : Préparation du (±)-2-(3-Chlorophényl)-2-oxo-3,3,5,5-tétraméthyl-[1,4,2]-oxazaphosphinane (3b)

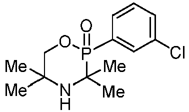

La synthèse de ce composé est notamment décrite dans l'article : Volle, J.-N., Kaloyanov, N., Saada, M. C., Virieux, D., Pirat, J.-L. Tetrahedron lett. 2007, 48, 4695-46972.

RMN-¹H (DMSO-d₆) δ 0,97 (3H, d, *J* = 16,5 Hz), 1,07 (3H, s), 1,23 (3H, d, *J* = 14,2 Hz), 1,29 (3H, s), 4,00 (1H, dd, *J* = 14,9 Hz, *J* = 11,1 Hz), 4,22 (1H, dd, *J* = 11,2 Hz, *J* = 5,7 Hz), 7,56 - 7,61 (1H, m), 7,70 - 7,73 (1H, m), 7,76 - 7,80 (2H, m). RMN-¹³C (DMSO-d₆) δ 26,93, 26,98, 27,03, 27,47 (s), 49,99 (d, *J* = 4,4 Hz), 50,17 (d, *J* = 92,2 Hz), 72,82 (d, *J* = 5,9 Hz), 130,53, 130,56, 130,62, 130,68, 131,13 (d, *J* = 10,2 Hz), 131,97 (d, *J* = 115,6 Hz), 132,30 (d, *J* = 2,9 Hz), 133,44 (d, *J* = 15,4 Hz). RMN-³¹P (DMSO-d₆) δ 35,35 (s). SM-HR ES+ : *m*/*z* calculé pour C₁₃H₂₀ClNO₂P [M+H]⁺ : 288,0920 ; trouvé : 288,0917.

### Procédure typique pour la préparation des 2-aryl-2-oxo-3,3,5,5-tétraméthyl-[1,4,2]-oxazaphosphinanes

A température ambiante, le bromure d'aryle ou l'iodure d'aryle (1,5 éq.), le tris(dibenzylidèneacétone)dipalladium(0) (Pd₂(dba)₃, 0,05 éq.), le 1,1'-bis(diphénylphosphino)ferrocène (dppf, 0,1 éq.) et la triéthylamine (3 éq.) sont successivement ajoutés à une solution de toluène (10,0 mL) contenant le 2-oxo-3,3,5,5-tétraméthyl-[1,4,2]-oxazaphosphinane (**5**, 2,46 mmol ou 2,82 mmol ; pour le composé **3i** : **5**, 3,89 mmol et toluène 13 mL). Le mélange réactionnel est alors agité et chauffé à 70°C durant toute la nuit. Après refroidissement, le mélange est filtré sur célite et la célite est lavée avec de l'acétate d'éthyle. Les filtrats sont réunis et concentrés sous vide. Le résidu obtenu est alors purifié.

### Exemple 2 : Préparation du (±)-2-(2-Chlorophényl)-2-oxo-3,3,5,5-tétraméthyl-[1,4,2]-oxazaphosphinane (3a)

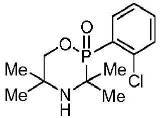

A partir du composé **5** (2,82 mmol) et du 1-bromo-2-chlorobenzène, et après purification sur colonne chromatographique C18, le produit **3a** est obtenu ; m = 144 mg, F. = 168,8- 169,9 °C. RMN-¹H (CDCl₃) δ 1,13 (3H, s), 1,16 (3H, d, *J* ∼ 18 Hz), 1,40 (3H, s), 1,48 (3H, d, *J* = 14,9 Hz), 1.82 (1H, s élargi), 4.01 (1H, dd, *J* = 15,2 Hz, *J* = 11,1 Hz), 4.48 (1H, dd, *J* = 11,2 Hz, *J* = 4,7 Hz), 7,34 - 7,39 (1H, m), 7,44 - 7,47 (2H, m), 7,98 - 8,02 (1H, m). RMN-¹³C (CDCl₃) δ 26,05 (d, *J* = 12,4 Hz), 26,65 (s), 28,24 (s), 28,53 (d, *J* = 2,9 Hz), 50,33 (d, *J* = 4,4 Hz), 51,97 (d, *J* = 90,0 Hz), 73.17 (d, *J* = 5,1 Hz), 126,66 (d, *J* = 9,5 Hz), 128,57 (d, *J* = 118,6 Hz), 131,14 (d, *J* = 8,0 Hz), 133,53 (d, *J* = 2,9 Hz), 135,45 (d, *J* = 5,1 Hz), 137,12 (d, *J* = 7,3 Hz). RMN-³¹P (CDCl₃) δ 35,08 (s). SM-HR ES+ : *m*/*z* calculé pour C₁₃H₂₀ClNO₂P [M+H]⁺ : 288,0920 ; trouvé : 288,0921.

### Exemple 3 : Préparation du (±)-2-(4-Chlorophényl)-2-oxo-3,3,5,5-tétraméthyl-[1,4,2]-oxazaphosphinane (3c)

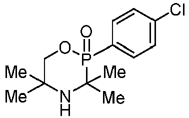

A partir du composé **5** (2,82 mmol) et du 4-chloro-1-iodobenzène, et après purification sur colonne chromatographique C18, le produit 3c est obtenu; m = 196 mg, F. = 139,8 - 140,4 °C. RMN-¹ H (CDCl₃) δ 1,10 (3H, d, *J* = 16,2 Hz), 1,14 (3H, s), 1,31 (3H, d, *J* = 14,4 Hz), 1,42 (3H, s), 1,76 (1H, s élargi), 3,99 (1H, dd, *J* = 15,5 Hz, *J* = 11,2 Hz), 4,48 (1H, dd, *J* = 4,6 Hz, *J* = 11,4 Hz), 7,45 - 7,48 (2H, m), 7,78 - 7,82 (2H, m). RMN-¹³C (CDCl₃) δ 26,66 (d, *J* = 11,0 Hz), 27,01 (s), 27,37 (d, *J* = 2,2 Hz), 28,48 (s), 50,59 (d, *J* = 5,1 Hz), 50,83 (d, *J* = 91,5 Hz), 73,53 (d, *J* = 5,1 Hz), 127,19 (d, *J* = 122,2 Hz), 128,98 (d, *J* = 12,4 Hz), 133,57 (d, *J* = 10,2 Hz), 139,29 (d, *J* = 2,9 Hz). RMN-³¹P (CDCl₃) δ 36,68 (s). SM-HR ES+ : *m*/*z* calculé pour C₁₃H₂₀NO₂PCl [M+H]⁺ : 288,0920; trouvé : 288,0920.

### Exemple 4 : Préparation du (±)-2-(3,5-Dichlorophényl)-2-oxo-3,3,5,5-tétraméthyl-[1,4,2]-oxazaphosphinane (3d)

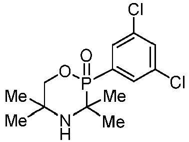

A partir du composé **5** (2,46 mmol) et du 1-bromo-3,5-dichlorobenzène, et après purification sur colonne chromatographique de gel de silice, le produit **3d** est obtenu ; m = 600 mg, F. = 177,0 - 177,8 °C. RMN-¹H (CDCl₃) δ 1,13 (3H, s), 1,13 (3H, d, *J* = 16,2 Hz), 1,32 (3H, d, *J* = 14,6 Hz), 1,42 (3H, s), 1,76 (1H, s élargi), 4,01 (1H, dd, *J* = 15,4 Hz, *J* = 11,4 Hz), 4,47 (1H, dd, *J* = 11,1 Hz, *J* = 3,3 Hz), 7,54 (1H, t, *J* = 1,8 Hz), 7,71 (2H, dd, *J* = 1,5 Hz, *J* = 10,4 Hz). RMN-¹³C (CDCl₃), δ 26,62 (d, *J* = 11,0 Hz), 27,03 (s), 27,34 (s), 28,41 (s), 50,60, 50,63 and 51,52, 73,87 (d, *J* = 4,4 Hz), 130,24 (d, *J* = 10,2 Hz), 132,65 (s), 132,74 (d, *J* = 116,4 Hz), 135,79 (d, *J* = 16,8 Hz). RMN-³¹P (CDCl₃) δ 34,93 (s); SM-HR ES+ : *m*/*z* calculé pour C₁₃H₁₉Cl₂NO₂P [M+H]⁺ : 322,0530 ; trouvé : 322,0533.

### Exemple 5 : Préparation du (±)-2-(2,3-Dichlorophényl)-2-oxo-3,3,5,5-tétraméthyl-[1,4,2]-oxazaphosphinane (3e)

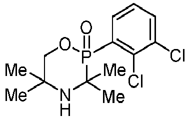

A partir du composé **5** (2,46 mmol) et du 1-bromo-2,3-dichlorobenzène, et après purification sur colonne chromatographique de gel de silice, le produit 3e est obtenu; m = 260 mg, F. = 163,8 - 164,5 °C. RMN-¹H (CDCl₃) δ 1,14 (3H, s), 1,16 (3H, d), 1,39 (3H, s), 1,49 (3H, d, *J* = 15,2 Hz), 1,81 (1H, s élargi), 4,01 (1H, dd, *J* = 15,2 Hz, *J* = 11,1 Hz), 4,48 (1H, dd, *J* = 11,1 Hz, *J* = 4,5 Hz), 7,31 (1H, td, *J* = 7,8 Hz, *J* = 2,8 Hz), 7,62 - 7,65 (1H, m), 7,93 (1H, ddd, *J* = 9,6 Hz, *J* = 7,8 Hz, *J* = 1,5 Hz). RMN-¹³C (CDCl₃) δ 26,09 (d, *J* = 12,4 Hz), 26,66 (s), 28,24 (s), 28,69 (d, *J* = 2,9 Hz), 50,43 (d, *J* = 5,1 Hz), 52,30 (d, *J* = 90,0 Hz), 73,58 (d, *J* = 5,1 Hz), 127,56 (d, *J* = 10,2 Hz), 131,39 (d, *J* = 114,9 Hz), 133,68 (d, *J* = 4,4 Hz), 134,38 (d, *J* = 2,2 Hz), 134,96, 135,08, 135,19, 135,27. RMN-³¹P (CDCl₃) δ 34,38 (s). SM-HR ES+ : *m*/*z* calculé pour C₁₃H₁₉Cl₂NO₂P [M+H]⁺ : 322,0530 ; trouvé : 322,0530.

### Exemple 6 : Préparation du (±)-2-(3-Fluorophényl)-2-oxo-3,3,5,5-tétraméthyl-[1,4,2]-oxazaphosphinane (3f)

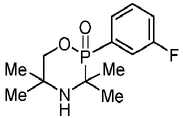

A partir du composé **5** (2,46 mmol) et du 1-bromo-3-fluorobenzène (48 h de chauffage à 70°C), et après purification sur colonne chromatographique de gel de silice, le produit **3f** est obtenu ; m = 213 mg, F. = 135,3 - 136,1 °C. RMN-¹H (CDCl₃) δ 1,12 (3H, d), 1,14 (3H, s), 1,33 (3H, d, *J* = 14,4 Hz), 1,42 (3H, s), 1,76 (1H, s élargi, N*H*), 4,00 (1H, dd, *J* = 15,4 Hz, *J* = 11,4 Hz), 4,49 (1H, dd, *J* = 11,4 Hz, *J* = 4,0 Hz), 7,23 - 7,28 (1H, m), 7,44 - 7,50 (1H, m), 7,53 - 7,58 (1H, m), 7,63 - 7,67 (1H, m). RMN-¹³C (CDCl₃) δ 26,67 (d, *J* = 11,0 Hz), 27,04 (s), 27,37 (d, *J* = 1,5 Hz), 28,47 (s), 50,63 (d, *J* = 4,4 Hz), 50,93 (d, *J* = 91,5 Hz), 73,59 (d, *J* = 4,4 Hz), 119,01 (dd, *J* = 22,3 Hz, *J* = 9,9 Hz), 119,78 (dd, *J* = 21,2 Hz, *J* = 2,2 Hz), 127,96 (dd, *J* = 8,8 Hz, *J* = 3,7 Hz), 130,56 (dd, *J* = 14,3 Hz, *J* = 7,7 Hz), 131,41 (dd, *J* = 119,3 Hz, *J* = 5,9 Hz), 162,56 (dd, *J* = 250,3 Hz, *J* = 16,8 Hz). RMN-³¹P (CDCl₃) δ 35,97 (s). RMN-¹⁹F (CDCl₃) δ -111,21 (s). SM-HR ES+ : *m*/*z* calculé pour C₁₃H₂₀FNO₂P [M+H]⁺ : 272,1216 ; trouvé : 272,1217.

### Exemple 7 : Préparation du (±)-2-(4-Fluorophényl)-2-oxo-3,3,5,5-tétraméthyl-[1,4,2]-oxazaphosphinane (3g)

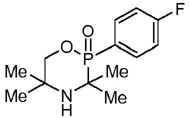

A partir du composé **5** (2,82 mmol) et du 1-bromo-4-fluorobenzène, et après purification sur colonne chromatographique C18, le produit **3g** est obtenu ; m = 227 mg, F. = 135,7 - 136,4 °C. RMN-¹H (CDCl₃) δ 1,10 (3H, d, *J* = 15,9 Hz), 1,14 (3H, s), 1,31 (3H, d, *J* = 14,4 Hz), 1,42 (3H, s), 1,76 (1H, s élargi), 3,99 (1H, dd, *J* = 15.4 Hz, *J* = 11.4 Hz), 4,49 (1H, dd, *J* = 11.4 Hz, *J* = 4,8 Hz), 7,15 - 7,20 (2H, m), 7,84 - 7,91 (2H, m). RMN-¹³C (CDCl₃) δ 26,64 (d, *J* = 11,0 Hz), 26,96 (s), 27,32 (d, *J* = 2,2 Hz), 28,42 (s), 50,52 (d, *J* = 4,4 Hz), 50,75 (d, *J* = 91,5 Hz), 73,46 (d, *J* = 4,4 Hz), 115,93 (dd, *J* = 21,2 Hz, *J* = 13,2 Hz), 124,64 (dd, *J* = 124,4 Hz, *J* = 3,7 Hz), 134,68 (dd, *J* = 10,2 Hz, *J* = 8,8 Hz), 165,52 (dd, *J* = 253,9 Hz, *J* = 3,7 Hz). RMN-³¹P (CDCl₃) δ 36,71 (s). RMN-¹⁹F (CDCl₃) δ -105,58 (s). SM-HR ES+ : *m*/*z* calculé pour C₁₃H₂₀FNO₂P [M+H]⁺ : 272,1216 ; trouvé : 272,1217.

### Exemple 8 : Préparation du (±)-2-(3-Nitrophényl)-2-oxo-3,3,5,5-tétramethyl-[1,4,2]-oxazaphosphinane (3h)

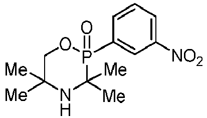

A partir du composé **5** (2,82 mmol) et du 1-bromo-3-nitrobenzène, et après purification sur colonne chromatographique sur gel de silice, le produit **3h** est obtenu ; m = 531 mg, F. = 131,9 - 132,7 °C. RMN-¹H (DMSO-d₆) δ 0,97 (3H, d, *J* = 16,7 Hz), 1,10 (3H, s), 1,27 (3H, d, *J* = 14,2 Hz), 1.31 (3H, s), 2.57 (1H, d élargi, *J* = 5,6 Hz), 4,05 (1H, dd, *J* = 15,0 Hz, *J* = 11,2 Hz), 4,26 (1H, dd, *J* = 11,1 Hz, *J* = 6,1 Hz), 7,85 (2H, td, *J* = 7,8 Hz, *J* = 3,0 Hz), 8,23 - 8,27 (1H, m), 8,45 - 8,54 (2H, m). RMN-¹³C (DMSO-d₆) δ 26,83 (d, *J* = 2,9 Hz), 27,00 (d, *J* = 9,5 Hz), 27,13 (s), 27,45 (s), 50,10 (d, *J* = 4,4 Hz), 50,30 (d, *J* = 93,0 Hz), 73,02 (d, *J* = 5,1 Hz), 126,39 (d, *J* = 10,2 Hz), 127,07 (d, *J* = 2,2 Hz), 130,49 (d, *J* = 11,7 Hz), 131,59 (d, *J* = 116,4 Hz), 138,20 (d, *J* = 8,8 Hz), 147,61 (d, *J* = 13,2 Hz). RMN-³¹P (DMSO-d₆) δ 35,41 (s). SM-HR ES+ : *m*/*z* calculé pour C₁₃H₂₀N₂O₄P [M+H]⁺ : 299,1161 ; trouvé : 299,1160.

### Exemple 9 : Préparation du (±)-2-(4-benzyloxycarbamoylphényl)-2-oxo-3,3,5,5-tétraméthyl-[1,4,2]-oxazaphosphinane (3i)

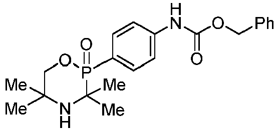

A partir du composé **5** (3,83 mmol) et du 4-bromo-phénylcarbamate de benzyle, et après purification sur colonne chromatographique sur gel de silice, le produit **3i** est obtenu ; m = 403 mg, F. = 196,1 - 197,0 °C. RMN-¹H (CDCl₃) δ 1,10 (3H, d, *J* ∼ 16,0 Hz), 1,12 (3H, s), 1,30 (3H, d, *J* = 14,3 Hz), 1,42 (3H, s), 2,06 (1H, s élargi, N*H*), 3,95 (1H, dd, *J* = 15,5 Hz, *J* = 11,4 Hz), 4,47 (1H, dd, *J* = 11,3 Hz, *J* = 4,3 Hz), 5,21 (s, 2H), 7,33 - 7,41 (5H, m), 7,52 - 7,54 (2H, m), 7,75 - 7,80 (2H, m). RMN-¹³C (CDCl₃) δ 26,51 (d, *J* = 11,0 Hz), 26,86 (s), 27,32 (d, *J* = 2,2 Hz), 28,28 (s), 50,51 (d, *J* = 4,4 Hz), 50,79 (d, *J* = 92,2 Hz), 67,07 (s), 73,27 (d, *J* = 5,1 Hz), 118,13 (dd, *J* = 12,4 Hz), 121,40 (dd, *J* = 127,3 Hz), 128,33 (s), 128,39 (s), 128,63 (s), 133,10 (d, *J* = 10,2 Hz), 135,99 (s), 142,84 (d, *J* = 2,9 Hz), 153,49 (s). RMN-³¹P (CDCl₃) δ 37,08 (s). SM-HR ES+ : *m*/*z* calculé pour C₂₁H₂₈N₂O₄P [M+H]⁺ : 403,1787 ; trouvé : 403,1789.

### Procédure générale pour la préparation des 2-hétéroaryl-2-oxo-3,3,5,5-tétraméthyl-[1,4,2]-oxazaphosphinanes

A température ambiante, le 2-oxyde-3,3,5,5-tétraméthyl-[1,4,2]-oxazaphosphinane [5] (**5**, 2,82 mmol), le bromure d'hétéroaryle (1,5 éq.), le tris(dibenzylidèneacétone)dipalladium(0) (Pd₂(dba)₃, 0,05 éq.), le 1,1'-bis(diphénylphosphino)ferrocène (dppf, 0.1 éq.) et la triéthylamine (3 éq. sauf pour **3l** 9 éq) sont ajoutés dans le toluène (10.0 mL). Le mélange réactionnel est alors agité et chauffé à 70°C toute la nuit. Après refroidissement, le mélange est filtré sur célite et la célite est lavé avec de l'acétate d'éthyle. Les filtrats sont réunis et concentrés sous vide. Le résidu obtenu est alors purifié.

### Exemple 10 : Préparation du (±)-2-(Pyridin-2-yl)-2-oxo-3,3,5,5-tétraméthyl-[1,4,2]-oxazaphosphinane (3j)

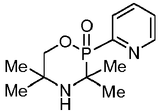

A partir de la 2-bromopyridine et après purification sur colonne chromatographique sur gel de silice, le produit 3j est obtenu ; m = 571 mg, F. = 87,6 - 88,2 °C. RMN-¹H (DMSO-d₆) δ 0,97 (3H, d, *J* = 16,7 Hz), 1,13 (3H, s), 1,20 (3H, s), 1,34 (3H, d, *J* = 13,4 Hz), 2,43 (1H, s élargi), 4,15 - 4,20 (2H, m), 7,58 - 7,62 (1H, m), 7,90 - 8,01 (2H, m), 8,79 - 8,80 (1H, m). RMN-¹³C (DMSO-d₆), δ 26,42 (d, *J* = 2,2 Hz), 26,65 (d, *J* = 9,5 Hz), 26,95 (s), 27,13 (s), 49,91 (d, *J* = 87,8 Hz), 50,02 (d, *J* = 5,9 Hz), 74,55 (d, *J* = 5,9 Hz), 126,17 (d, *J* = 2,9 Hz), 127,60 (d, *J* = 19,0 Hz), 136,62 (d, *J* = 8,8 Hz), 149,97 (d, *J* = 19,0 Hz), 153,86 (d, *J* = 146,4 Hz). RMN-³¹P (DMSO-d₆) δ 29,13 (s). SM-HR ES+ : *m*/*z* calculé pour C₁₂H₂₀N₂O₂P, [M+H]⁺ : 255,1262 ; trouvé: 255,1264.

### Exemple 11 : Préparation du (±)-2-(Pyridin-3-yl)-2-oxo-3,3,5,5-tétraméthyl-[1,4,2]-oxazaphosphinane (3k)

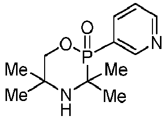

A partir de la 3-bromopyridine et après purification sur colonne chromatographique sur gel de silice, le produit **3k** est obtenu ; m = 504 mg, F. = 156,4 - 157,9 °C. RMN-¹H (DMSO-d₆) δ 0,97 (3H, d, *J* = 16,7 Hz), 1,08 (3H, s), 1,24 (3H, d, *J* = 14,1 Hz), 1,30 (3H, s), 4,00 (1H, dd, *J* = 15,0 Hz, *J* = 11,2 Hz), 4,24 (1H, dd, *J* = 11,2 Hz, *J* = 5,9 Hz), 7,55 - 7,59 (1H, m), 8,16 - 8,22 (1H, m), 8,79 - 8,81 (1H, m), 8,92 - 8,93 (1H, m). RMN-¹³C (DMSO-d₆) δ 26,84 (d, *J* = 2,9 Hz), 27,01 (s), 27,02 (d, *J* = 10,2 Hz), 27,47 (s), 50,01 (d, *J* = 4,4 Hz), 50,17 (d, *J* = 92,9 Hz), 72,67 (d, *J* = 5,1 Hz), 123,71 (d, *J* = 8,8 Hz), 125,46 (d, *J* = 115,6 Hz), 139,89 (d, *J* = 8,1 Hz), 151,90 (d, *J* = 11,0 Hz), 152,90 (s). RMN-³¹P (DMSO-d₆) δ 35,37 (s). SM-HR ES+ : *m*/*z* calculé pour C₁₂H₂₀N₂O₂P, [M+H]⁺ : 255,1262 ; trouvé : 255,1264.

### Exemple 12 : Préparation du (±)-2-(Pyridin-4-yl)-2-oxo-3,3,5,5-tétraméthyl-[1,4,2]-oxazaphosphinane (3l)

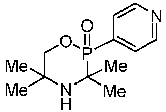

A partir de la 4-bromopyridine hydrochlorée et après purification sur colonne chromatographique sur gel de silice, le produit **3l** est obtenu ; m = 354 mg, F. = 152,2 - 153,1 °C. RMN-¹H (DMSO-d₆) δ 0,97 (3H, d, *J* = 16,7 Hz), 1,08 (3H, s), 1,25 (3H, d, *J* = 14,4 Hz), 1,29 (3H, s), 4,02 (1H, dd, *J* = 14,8 Hz, *J* = 11,2 Hz), 4,23 (1H, dd, *J* = 11,4 Hz, *J* = 6,1 Hz), 7,73 - 7,77 (2H, m), 8,76 - 8,79 (2H, m). RMN-¹³C (DMSO-d₆) δ 26,81, 26,89, 26,91, 27,39, 49,99 (d, *J* = 5,1 Hz), 50,06 (d, *J* = 91,5 Hz), 72,92 (d, *J* = 5,1 Hz), 125,66 (d, *J* = 8,0 Hz), 138,00 (d, *J* = 112,0 Hz), 149,87 (d, *J* = 9,5 Hz). RMN-³¹P (DMSO-d₆) δ 34,55 (s). SM-HR ES+: *m*/*z* calculé pour C₁₂H₂₀N₂O₂P [M+H]⁺ : 255,1262 ; trouvé : 255,1265.

### Exemple 13 : Préparation du (±)-2-(Pyrimidin-2-yl)-2-oxo-3,3,5,5-tétraméthyl-[1,4,2]-oxazaphosphinane (3m)

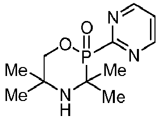

A partir de la 2-bromopyrimidine et après purification sur colonne chromatographique sur gel de silice, le produit **3m** est obtenu ; m = 594 mg, F. = 116,1 - 117,2 °C. RMN-¹H (DMSO-d₆) δ 1,08 (3H, d, *J* = 16,4 Hz), 1,14 (6H, s), 1,38 (3H, d, *J* = 13,6 Hz), 4,19 (2H, d, *J* = 9,9 Hz), 7,69 (1H, td, *J* = 4,9 Hz, *J* = 3,3 Hz), 8,99 (2H, dd, *J* = 5,1 Hz, *J* = 0,8 Hz). RMN-¹³C (DMSO-d₆) δ 26,32, 26,43, 26,45, 26,78, 27,08, 49,88 (d, *J* = 5,1 Hz), 50,14 (d, *J* = 88,6 Hz), 75,44 (d, *J* = 6,6 Hz), 123,00 (d, *J* = 2,9 Hz), 156,99 (d, *J* = 13,9 Hz), 164,69 (d, *J* = 176,4 Hz). RMN-³¹P (DMSO-d₆) δ 26,62 (s). SM-HR ES+ : *m*/*z* calculé pour C₁₁H₁₉N₃O₂P [M+H]⁺ : 256,1215 ; trouvé : 256,1217.

### Exemple 14 : Préparation du (±)-2-(Pyrimidin-5-yl)-2-oxo-3,3,5,5-tétraméthyl-[1,4,2]-oxazaphosphinane (3n)

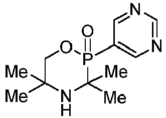

A partir de la 5-bromopyrimidine et après purification sur colonne chromatographique sur gel de silice, le produit **3n** est obtenu ; m = 600 mg, F. = 140,2 - 141,1 °C. RMN-¹H (DMSO-d₆) δ 1,03 (3H, d, *J* = 17,2 Hz), 1,09 (3H, s), 1,29 (3H, d, *J* = 14,4 Hz), 1,29 (3H, s), 2,63 (1H, d élargi, *J* = 4,8 Hz), 4,04 (1H, dd, *J* = 14,9 Hz, *J* = 11,4 Hz), 4,24 (1H, dd, *J* = 11,4 Hz, *J* = 6,3 Hz), 9,14 (2H, d, *J* = 4,8 Hz), 9,42 (1H, d, *J* = 3,0 Hz). RMN-¹³C (DMSO-d₆) δ 26,55 (d, *J* = 3,7 Hz), 26,81 (d, *J* = 10,2 Hz), 27,13 (s), 27,44 (s), 50,03 (d, *J* = 4,4 Hz), 50,30 (d, *J* = 92,9 Hz), 72,86 (d, *J* = 5,9 Hz), 124,03 (d, *J* = 113,4 Hz), 159,87 (d, *J* = 9,5 Hz), 160,86 (s). RMN-³¹P (DMSO-d₆) δ 33,82 (s). SM-HR ES+ : *m*/*z* calculé pour C₁₁H₁₉N₃O₂P [M+H]⁺ : 256,1215; trouvé: 256,1216.

### Exemple 15 : Préparation du (±)-2-(3-aminophényl)-2-oxo-3,3,5,5-tétraméthyl-[1,4,2]-oxazaphosphinane (6)

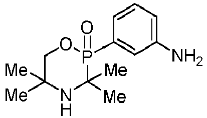

A température ambiante et sous une pression atmosphérique d'hydrogène, le composé **3h** (1.19 mmol) et le palladium (10%)/C (0.025 g) sont agités dans l'acétate d'éthyle (5,5 mL) durant une nuit. Le mélange réactionnel est alors filtré sur célite et la célite est rincée avec le dichlorométhane. Les filtrats sont réunis et concentrés. Le brut obtenu est purifié par colonne chromatographique sur gel de silice pour donner le produit 6; m = 191 mg, F. = 140,3 - 141,1 °C. RMN-¹H (DMSO-d₆) δ 0,96 (3H, d, *J* = 15,7 Hz), 1,03 (3H, s), 1,16 (3H, d, *J* = 13,9 Hz), 1,28 (3H, s), 2,28 (1H, s élargi), 3,91 (1H, dd, *J* = 14,5 Hz, *J* = 11,2 Hz), 4,19 (1H, dd, *J* = 10,9 Hz, *J* = 5,1 Hz), 5,36 (2H, s), 6,73 - 6,75 (1H, m), 6,85 - 6,90 (1H, m), 7,00 - 7,03 (1H, m), 7,11 - 7,16 (1H, m). RMN-¹³C (DMSO-d₆) δ 26,73, 27,00, 27,12, 27,52, 49,98 (d, *J* = 3,7 Hz), 50,01 (d, *J* = 90,2 Hz), 72,37 (d, *J* = 5,1 Hz), 116,64 (d, *J* = 10,3 Hz), 117,34 (d, *J* = 2,2 Hz), 118,64 (d, *J* = 9,5 Hz), 128,93 (d, *J* = 13,9 Hz), 129,41 (d, *J* = 119,6 Hz), 148,65 (d, *J* = 13,9 Hz). RMN-³¹P (DMSO-d₆) δ 37,11 (s). SM-HR ES+ : *m*/*z* calculé pour C₁₃H₂₂N₂O₂P [M+H]⁺ : 269,1419 ; trouvé : 269,1420.

### Exemple 16 : Préparation du (±)-2-(4-aminophényl)-2-oxo-3,3,5,5-tétraméthyl-[1,4,2]-oxazaphosphinane (7)

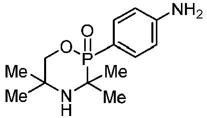

A température ambiante et sous une pression atmosphérique d'hydrogène, le composé **3i** (0.75 mmol) et le palladium (10%)/C (0.017 g) sont agités dans le méthanol (3,5 mL) durant toute la nuit. Le mélange réactionnel est filtré sur célite et la célite est rincée avec le dichloromethane. Les filtrats sont réunis et concentrés. Le brut obtenu est purifié par colonne chromatographique sur gel de silice pour donner le produit **7** ; m = 160 mg, F. = 160,3 - 161,1 °C. RMN-¹H (CDCl₃) δ 1,10 (3H, s), 1,11 (3H, d, *J* = 15,7 Hz), 1,27 (3H, d, *J=* 14,2 Hz), 1,40 (3H, s), 1,75 (1H, s élargi), 3,93 (1H, dd, *J* = 15,5 Hz, *J* = 11,3 Hz), 4,07 (2H, s élargi), 4,46 (1H, dd, *J* = 11,3 Hz, *J* = 4,7 Hz), 6,68 (2H, dd, *J* = 8,4 Hz, *J* = 2,6 Hz), 7,60 (2H, dd, *J* = 10,3 Hz, *J* = 8,4 Hz). RMN-¹³C (CDCl₃) δ 26,77 (d, *J* = 11,0 Hz), 27,03 (s), 27,43 (d, *J* = 2,2 Hz), 28,52 (s), 50,54 (d, *J* = 5,1 Hz), 50,89 (d, *J* = 92,4 Hz), 73,25 (d, *J* = 4,4 Hz), 114,28 (d, *J* = 13,2 Hz), 116,05 (d, *J* = 132,0 Hz), 133,91 (d, *J* = 11,0 Hz), 150,52 (dd, *J* = 2,9 Hz). RMN-³¹P (CDCl₃) δ 38,17 (s). SM-HR ES+ : *m*/*z* calculé pour C₁₃H₂₂N₂O₂P [M+H]⁺ : 269,1419 ; trouvé : 269,1419.

### Exemple 17 : Préparation du (±)-2-(3-chlorophényl)-N-méthyl-2-oxo-3,3,5,5-tétraméthyl-[1,4,2]-oxazaphosphinane (8)

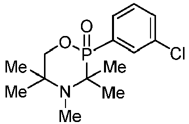

Le composé **3b** (1.74 mmol), le carbonate de potassium (3.47 mmol) et l'iodure de méthyle (3.47 mmol) sont ajoutés dans l'acétone (7.0 mL), puis le mélange est chauffé à reflux durant 3 jours. Après refroidissement, le solvant est évaporé sous vide. Le brut obtenu est alors purifié par colonne chromatographique sur gel de silice pour donner le produit **8** ; m = 270 mg, F. = 144,9 - 145,5 °C. RMN-¹H (DMSO-d₆) δ 0,89 (3H, d, *J* = 17,9 Hz), 1,04 (3H, s), 1,25 (3H, s), 1,36 (3H, d, *J* = 12,6 Hz), 2,19 (3H, s), 3,97 (1H, dd, *J* = 20,5 Hz, *J* = 12,4 Hz), 4,34 (1H, dd, *J* = 12,1 Hz, *J* = 9,1 Hz), 7,56 - 7,61 (1H, m), 7,70 - 7,73 (1H, m), 7,80 - 7,85 (2H, m). RMN-¹³C (DMSO-d₆) δ 19,42 (d, *J* = 2,9 Hz), 21,44 (s), 25,23 (s), 25,31 (s), 26,91 (d, *J=* 11,7 Hz), 53,98 (d, *J* = 108,3 Hz), 54,95 (s), 71,50 (d, *J* = 5,1 Hz), 130,54 (d, *J* = 13,2 Hz), 131,31 (d, *J* = 8,1 Hz), 131,69 (d, *J* = 126,6 Hz), 131,95 (d, *J* = 9,5 Hz), 132,53 (d, *J* = 2,2 Hz), 133,26 (d, *J* = 15,4 Hz). RMN-³¹P (DMSO-d₆) δ 43,21 (s). SM-HR ES+: *m*/*z* calculé pour C₁₄H₂₂ClNO₂P [M+H]⁺ : 302,1077 ; trouvé : 302,1053.

### Exemple 18 : Préparation du (±)-2-(3-chlorophényl)-2-thiono-3,3,5,5-tétraméthyl-[1,4,2]-oxazaphosphinane (9)

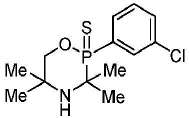

Sous une atmosphère d'azote, le 2-(3-dichlorophényl)-3,3,5,5-tétraméthyl-2-oxo-[1,4,2]-oxazaphosphinane (**3b**, 3.48 mmol), le réactif de Lawesson (3.49 mmol) et le toluène (17 mL) sont agités et chauffés à 95 °C durant 21 h. La solution est alors refroidie et le surnageant est récupéré. La pâte restante est rincée avec du toluène, et les phases organiques sont réunies et évaporées. Le résidu obtenu est purifié sur colonne chromatographique sur gel de silice pour donner le produit 9 ; m = 171 mg, F. = 103,9 - 104,8 °C. RMN-¹H (CDCl₃) δ 1,13 (3H, s), 1,18, 1,22, 1,23 et 1,27 (6H), 1,42 (3H, s), 1,99 (1H, s élargi), 3,94 (1H, dd, *J* = 18,6 Hz, *J* = 11,2 Hz), 4,64 (1H, dd, *J* = 11,2 Hz, *J* = 6,9 Hz), 7,39 - 7,44 (1H, m), 7,49 - 7,52 (1H, m), 7,78 - 7,83 (1H, m), 7,88 - 7,92 (1H, m). RMN-¹³C (CDCl₃) δ 25,81 (d, *J* = 10,3 Hz), 27,39 (s), 28,77 (s), 28,96 (d, *J* = 8,1 Hz), 50,35 (d, *J* = 4,4 Hz), 54,38 (d, *J* = 61,6 Hz), 72,45 (d, *J* = 5,1 Hz), 129,69 (d, *J* = 10,3), 129,91 (d, *J* = 13,9 Hz), 131,30 (d, *J* = 11,0 Hz), 132,33 (d, *J* = 2,9 Hz), 134,25 (d, *J* = 92,4 Hz), 134,94 (d, *J* = 16,1 Hz). RMN-³¹P (CDCl₃) δ 90,12 (s). SM-HR ES+: *m*/*z* calculé pour C₁₃H₂₀ClNOPS [M+H]⁺ : 304,0692 ; trouvé : 304,0694.

### ACTIVITÉ DES COMPOSÉS

### A. MATERIELS ET METHODES

### 1. Animaux

Des souris males (RjOrl:SWISS) des laboratoires Janvier (Le Genest-Saint-Isle, France), âgées de 7-9 semaines et pesant 32 ± 2 g ont été utilisées pour cette étude. Les animaux ont été groupés dans des cages en plastique, avec un accès libre à la nature et l'eau, dans un environnement régulé (23 ± 1°C, humidité 40-60%, 12 h cycle lumière/obscurité).

### 2. Composés et peptides

Les composés sont solubilisés dans une solution saline physiologique (NaCl 0.9%) ou dans du diméthylsulfoxyde (DMSO) 10% dans la solution saline et administrés par voie intraperitonéale (IP) dans un volume de 100 µl pour 20 g de poids corporel.

Les peptides amyloid-β[25-35] (Aβ₂₅₋₃₅) et contrôle (Sc.Aβ) viennent de Genepep (Saint-Jean-de-Védas, France). Ils sont solubilisés dans de l'eau distillée injectable à la concentration de 3 mg/ml et conservés à -20°C. Avant l'injection, les peptides sont incubés à 37°C pendant 4 jours, ce qui permet au peptide Aβ₂₅₋₃₅, mais pas au Sc.Aβ, de former des oligomères. Ils sont administrés par voie intracérébroventriculaire (ICV). Les animaux sont anesthésiés par inhalation d'isoflurane 2,5% (TEM) et reçoivent 3 µl de solution de peptide par voie ICV directe (méthode de Haley et McCormick). L'injection se fait avec une micro-seringue Hamilton de 10 µl à un débit de 1 µl/min.

### 3. Alternance spontanée dans le labyrinthe en Y

Les animaux ont été testés pour leur performance d'alternance spontanée dans le labyrinthe en Y, un indice de la mémoire spatiale de travail. Le labyrinthe en Y est en PVC gris. Chaque bras est long de 40 cm, haut de 13 cm, large de 3 cm à la base et de 10 cm à la partie supérieure, et convergeant à un angle égal. Chaque souris est placée à l'extrémité d'un bras et peut se déplacer librement à travers le labyrinthe lors d'une session de 8 min. La série d'entrées dans les bras, y compris les éventuels retours dans le même bras, est analysée visuellement. Une alternance est définie comme étant les entrées consécutives dans trois bras différents. Le nombre d'alternances maximal sera donc le nombre total d'entrées dans les branches moins deux et le pourcentage d'alternance calculé comme (alternances réelles / alternances maximales) x 100. Les paramètres comprennent le pourcentage d'alternance (indice de mémoire) et le nombre total d'entrées de bras (indice d'exploration).

### 4. Test d'évitement passif

L'appareil est à deux compartiments (15 × 20 × 15 cm de haut) avec une partie éclairée avec des murs en PVC blanc et l'autre sombre avec des murs en PVC noir et un sol grillagé. Une porte guillotine sépare chaque compartiment. Une lampe de 60 W est placée 40 cm au-dessus du compartiment blanc pendant l'expérience. Des chocs électriques (0,3 mA pendant 3 s) peuvent être délivrés à la grille à l'aide d'un générateur de choc brouillés (Lafayette Instruments, Lafayette, Etats-Unis). La porte guillotine est d'abord fermée pendant la session d'entrainement. Chaque souris est placée dans le compartiment blanc. Après 5 s, la porte est ouverte. Lorsque la souris pénètre dans le compartiment sombre et place ses pattes sur le sol de la grille, la porte est fermée et le choc électrique délivré pendant 3 s. La latence de passage (STL), qui est le temps mis pour entrer dans le compartiment sombre, et le nombre de cris est enregistré. Le test de rétention est effectué après 24 h. Chaque souris est à nouveau placée dans le compartiment blanc. Après 5 s, la porte est ouverte. La latence de passage est enregistrée jusqu'à 300 s.

### 5. Apprentissage spatial en piscine

La piscine est un bassin circulaire (diamètre 140 cm, hauteur 40 cm). La température de l'eau, 22-24°C, l'intensité de la lumière, les repères externes dans la pièce, et l'opacité de l'eau sont rigoureusement reproduits. Une plate-forme transparente en plexiglas non glissante (diamètre 10 cm) est immergée sous la surface de l'eau lors de l'acquisition. Les nages peuvent être enregistrées en utilisant un logiciel Videotrack® ViewPoint, Champagne-au-Mont-d'Or, France), avec les trajectoires analysées en durée et distances. Le logiciel divise la piscine en quatre quarts.

Phase d'acquisition : Elle consiste en 3 nages par jour pendant 5 jours, à 20 min d'intervalle. Les positions de départ sont fixées aux points cardinaux délimitant les quarts, et choisies aléatoirement. Chaque animal a 90 s pour trouver la plate-forme. La latence de nage est mesurée. Les animaux sont laissés sur la plate-forme pendant 20 s. Les animaux qui n'ont pas trouvé la plate-forme après 90 s y sont placés manuellement et laissé pendant 20 s. La médiane des durées de nage est calculée pour chaque jour et exprimée pour le groupe expérimental en moyenne ± SEM.

Phase de rappel : un test d'essai est réalisé 24 h après la dernière session d'acquisition. La plate-forme est enlevée et chaque animal nage pendant 60 s. La session est suivie par vidéo et le temps passé dans le quart d'entrainement (où était placée la plate-forme) est mesuré.

### 6. Test de reconnaissance d'objet

L'appareil consiste en quatre arènes carrées (50 cm x 50 cm x 50 cm de haut) faites en plexiglas blanc et placées sur un plateau équipé de diodes infrarouges électroluminescentes (IR). L'activité locomotrice de l'animal et la position de leur nez peuvent être capturées par une caméra sensible à l'IR et elles peuvent être analysées en utilisant les logiciels Videotrack et Nosetrack. Le premier jour, les animaux sont acclimatés pendant 10 min à l'open-field. Au jour 2, deux objets identiques (flacons en plastique avec bouchon) ont été placés à des positions définies, à ¼ et ¾ d'une diagonale de l'arène. Chaque souris est placée dans le champ-ouvert et l'activité exploratoire et la position du nez est enregistrée durant 10 min. L'activité est analysée en termes de nombre de contact avec les objets et de durée des contacts. Au jour 3, l'objet en position 2 est remplacé par une nouveauté (protection de pieds de chaise en plastique noir) différant en forme, couleur et texture de l'objet familier. Chaque souris est placée à nouveau dans le champ-ouvert et l'activité exploratoire enregistrée pendant 10 min. L'activité est analysée de façon similaire. L'indice d'exploration préférentiel est calculé comme le rapport entre le nombre (ou la durée) des contacts avec l'objet en position 2 sur le nombre total (ou la durée) des contacts avec les deux objets. Les animaux qui présentent moins de 10 contacts avec des objets pendant les séances 2 et 3 sont généralement exclus des calculs.

### 7. Mesures du stress oxydant

Après sacrifice, les hippocampes et cortex des cerveaux des souris sont disséqués et congelés à -80°C jusqu'à leur utilisation. L'accumulation des espèces réactives de l'oxygène a été mesurée par fluorescence de la 2',7'-dichlorofluorescein (DCF) dans l'hippocampe des souris *ex vivo.* Le diacétate de DCF (0,5 µM) (Sigma-Aldrich) est ajouté dans des fractions SDS solubles d'extraits d'd'hippocampe. Après 30 min à 37°C, la fluorescence du DCF est quantifiée (excitation à 485 nm, émission à 530 nm) dans un spectrofluorimètre Fluoroskan Ascent (Thermo Scientific, Waltham, USA), et normalisée avec les concentrations en protéine des extraits.

La peroxydation des lipides membranaires est mesurée par la méthode du cumène/xylenol. Les hippocampes sont homogénéisés dans le méthanol (1/10), centrifugés à 1 000 g pendant 5 min et le surnageant collecté. Des aliquotes sont ajoutés à une solution contenant du FeSO₄ 1mM, H₂SO₄ 0.25 M, xylenol orange 1 mM et incubés pendant 30 min à température ambiante. L'absorbance est mesurée à 580 nm (A₅₈₀1), et 10 µl de cumène hydroperoxide (CHP) 1 mM est ajouté et l'échantillon est incubé 30 min à température ambiante. L'absorbance est mesurée à 580 nm (A₅₈₀2). Le niveau de peroxydation des lipides est déterminé comme CHP équivalents selon : CHP eq. = A₅₈₀1/A₅₈₀2 x [CHP (nmol)] x dilution, et calculé en CHP eq. par poids de tissue, exprimé en % du groupe contrôle.

### 8. Mesure des taux d'expression de Bax, Bcl-2, TNFα, IL-1β et synaptophysine

Les hippocampes ont été homogénéisés dans une solution 50 mM Tris-150 mM NaCl, pH 7.5, et soniqués pendant 20 s. Après centrifugation à 16 100 g pendant 15 min à 4°C, les surnageants sont utilisés pour les dosages ELISA selon les instructions du fabricant (ThermoScientific, Courtaboeuf, France ; USCN, Wuhan, Chine). Dans chaque essai, l'absorbance est lue à 450 nm et la concentration de chaque échantillons calculée par comparaison avec une courbe étalon. Les résultats sont exprimés en ng ou pg par mg de tissu et présentés en % du groupe contrôle.

### 19. Analyses statistiques

Les données ont été analysées par analyse de variance à un facteur (ANOVA, valeur F), suivie d'un test de comparaison multiple de Dunnett. Les durées de nage ne suivent pas une distribution gaussienne puisqu'une valeur maximale est posée. Les profils d'acquisition ont donc été analysés par une ANOVA non-paramétrique en mesure répétée de Friedman, suivie d'un test de Dunn ou de Mann-Whitney. Les données du test d'essai sont présentées en temps de présence dans les quarts d'entrainement (T) ou dans les trois autres quarts en moyenne (o) et analysés par un test t par rapport au niveau de hasard (15 s). La préférence d'objet, calculée à partir du nombre de contacts ou de la durée de contact avec les objets, a été analysée avec un test t par rapport au niveau de hasard (50%). Le niveau de signification statistique est p < 0.05.

### B. RESULTATS PHARMACOLOGIQUES

### 1. Analyse de la protection induite par le composé 3b

Le composé **3b** est injecté par voie IP, de 0,3 à 3 mg/kg, immédiatement avant l'injection ICV de peptide Aβ₂₅-₃₅ oligomérisé, modèle pharmacologique aigu de toxicité amyloïde. Après une semaine, les animaux sont testés au niveau comportemental, puis sacrifiés, le cerveau disséqué pour des analyses biochimiques.

Au jour 7 après les injections d'**3b** et de peptide amyloïde, les souris ont été testées pour leur capacité d'alterner dans le labyrinthe en Y, un test de mémoire spatiale de travail. Par rapport aux animaux contrôles, ayant reçu en ICV un peptide non toxique (scrambled Aβ, ScAβ) et qui montrent un pourcentage d'alternance de 65% (Fig. 1a), les animaux Aβ₂₅₋₃₅ montrent un déficit très significatif. L'administration de composé **3b** prévient ce déficit, significativement aux doses de 0,7, 1,5 et 3 mg/kg (Fig. 1a). Les traitements n'affectent pas les capacités de mobilité et d'exploration des animaux, puisque le nombre total de bras explorés durant la session de 8 min, ne changent pas (Fig. 1b). Les souris ont été ensuite testées pour leur réponse d'évitement passif, un test de mémoire non-spatiale à long-terme, avec entrainement au jour 8 et rappel au jour 9 après les injections (Fig. 1c). Les animaux Aβ₂₅₋₃₅ montrent un déficit très significatif de la réponse. Toutes les doses testées d'3b ont permis de prévenir ce déficit (Fig. 1c).

La dose de 0,7 mg/kg étant la dose minimale active dans ces expériences de dose-dépendance, un groupe a été injecté et testé aux jours 7 à 9 dans une procédure de reconnaissance d'objet. La session 1 consiste en une habituation à l'arène carrée dans laquelle se fait le test. La session 2 consiste à placer 2 objets identiques dans l'arène et à mesurer le temps d'interaction et le nombre de contacts de l'animal avec les objets. La session 3 consiste à remplacer l'un des 2 objets par un nouvel objet et à mesurer la préférence de l'animal pour ce nouvel objet. Chaque session est séparée de 24 h. Les résultats sont présentés en nombre de contacts (Fig. 1d) ou en durée d'interaction (Fig. 1e). Dans les 2 cas, on note que les souris contrôles ScAβ ne montrent pas de préférence entre les 2 objets identiques lors de la session 2, mais montrent une préférence significative pour le nouvel objet lors de la session 3 (Fig. 1d, e). Les animaux Aβ₂₅₋₃₅ ne montrent pas de préférence, par contre, le traitement avec le composé **3b** restaure une préférence significative. Ces résultats montrent que le composé **3b** protège la mémoire de reconnaissance, altérée dans le modèle. Enfin, on notera que dans tous les cas, le composé **3b**, à 0,7 ou 3 mg/kg, n'affecte pas les performances des animaux contrôles ScAβ.

Au jour 9, les animaux ont été sacrifiés, les cerveaux prélevés, les hippocampes et cortex disséqués et congelés. Les analyses biochimiques ont consisté à mesurer l'ampleur du stress oxydant, l'induction des voies apoptotiques, la neuroinflammation, les altérations synaptiques et le tonus cholinergique, chez les animaux, soit par des tests colorimétriques ou radioactifs, soit en utilisant des kits Elisa commerciaux. Un stress oxydant est très significativement induit après injection de peptide Aβ₂₅₋₃₅, ce que l'on a mesuré par une augmentation des espèces réactives de l'oxygène (ROS) dans l'hippocampe (test de fluorescence du DCF, Fig. 2a), soit par une augmentation du niveau de peroxydation des lipides membranaires (LPO) dans le cortex (Fig. 2b). Le traitement à l'**3b**, notamment à la dose de 0,7 mg/kg IP, a permis de très significativement bloquer l'induction des ROS (Fig. 2a), soit plus efficacement de diminuer le niveau de LPO (Fig. 2b).

L'induction des voies de mort programmée des cellules, l'apoptose, se mesure par divers marqueurs. La voie dite intrinsèque peut se mesurer par une augmentation de la protéine pro-apoptotique Bax et/ou une diminution de la protéine anti-apoptotique Bcl2, et donc une augmentation du rapport Bax/Bcl2. Nous avons observé une augmentation significative de Bax (Fig. 2c) et pas de changement de Bcl2 (Fig. 2d) après injection du peptide Aβ₂₅₋₃₅. Mais le rapport Bax/Bcl2 augmente bien significativement (Fig. 3e). Le traitement avec **3b** dans la gamme de dose 0,3-1,5 mg/kg IP diminue significativement l'augmentation de Bax (Fig. 2c) et donc le rapport Bax/Bcl2 (Fig. 2e). Il faut noter que le composé **3b**, testé à 1,5 mg/kg IP, tend à augmenter Bax (Fig. 2c) et donc le rapport Bax/Bcl2 (Fig. 2e) chez les animaux contrôle ScAβ. Le traitement est sans effet sur les taux de Bcl2 (Fig. 2d).

La neuroinflammation a été analysée en mesurant les taux tissulaires de 2 cytokines pro-inflammatoires, le TNFα (Fig. 3a) et l'IL6 (Fig. 3b), dont on sait qu'elles sont augmentées dans le modèle. L'augmentation très significative de TNFα est prévenue par le composé **3b** à la dose de 0,7 mg/kg IP (Fig. 3a). L'augmentation significative d'IL6 est atténuée, mais de façon non-significative, par la même dose de **3b** (Fig. 5b).

Les altérations synaptiques ont été évaluées par mesure de l'expression de la protéine de d'échafaudage pré-synaptique synaptophysine (Fig. 3c). Une diminution très significative de 20% est mesurée, qui est prévenue par **3b**, à la dose de 0,7 mg/kg IP (Fig. 3c).

Le tonus cholinergique a été évalué par une mesure de l'activité de la choline acétyltransférase, l'enzyme limitant la synthèse de l'acétycholine. On observe une diminution modérée mais très significative de l'activité enzymatique dans le modèle, qui est bloquée par le traitement avec **3b**, 0,7 mg/kg Ip (Fig. 3d).

A partir du criblage des molécules de la série (voir plus loin), le composé **3c**, isomère de position du Chlore de **3b**, s'est avéré très efficace. Il a donc été testé dans une gamme de dose étendue, de 0,03 à 0,7 mg/kg IP, dans le modèle Aβ₂₅₋₃₅. Les animaux ont été testés en alternance spontanée au jour 7, en évitement passif aux jours 8 et 9 (Fig. 4). L'analyse a confirmé que la dose de 0,3 mg/kg IP prévient de façon significative les déficits d'alternance spontanée induits par Aβ₂₅-₃₅ (Fig. 4a), mais cette dose seule est apparue efficace. En parallèle, on a mesuré un effet significatif d'hyperactivité sur le nombre total de bras du labyrinthe explorés durant les 8 min de la session (Fig. 4b). Les doses de 0,1 et 0,3 mg/kg IP sont apparues efficace sur la réponse de mémoire à long-terme (Fig. 4c).

Enfin, une première analyse mécanistique de l'effet pharmacologique *in vivo* du composé **3b** a été menée. Dans le but de confirmer que le composé, neuroprotecteur contre la toxicité amyloïde, pouvait également être anti-amnésiant dans un modèle pharmacologique d'amnésie, nous avons tout d'abord établi son efficacité contre les effets amnésiants de la scopolamine. Des animaux ont été traités avec le composé **3b**, 0,03-1,5 mg/kg lp, 10 min avant de recevoir de la scopolamine, 0,5 mg/kg SC, un antagoniste des récepteurs muscariniques cholinergiques. La scopolamine a été injectée 20 min avant une tâche mnésique : une mesure de l'alternance spontanée dans le labyrinthe en Y (Fig. 5a, b) ou un entrainement au test d'évitement passif (Fig. 5c, d). Il est apparu que le composé **3b** atténue de façon significative les déficits d'alternance induits par la scopolamine, aux doses de 0,1 et 0,3 mg/kg IP (Fig. 5a). En parallèle, aux doses les plus faibles testées, il semble augmenter l'effet hyper locomoteur de la scopolamine (Fig. 5b). Sur le test de mémoire à long-terme, le composé atténue la diminution de latence de passage induite par la scopolamine, aux mêmes doses de 0,1 et 0,3 mg/kg (Fig. 5c) et diminue l'augmentation de la latence d'échappement induite par la scopolamine, à toutes les doses testées (Fig. 5d). Le composé est bien anti-amnésiant et la dose efficace est 0,1 mg/kg IP.

Les résultats du profilage CEREP ont suggéré une augmentation de la fixation du radioligand α₇ nicotinique. L'effet d'une co-administration d'antagonistes des récepteurs nicotiniques cholinergiques et σ₁ a été examiné sur l'effet anti-amnésiant du composé **3b**, 0.1 mg/kg IP, soit la méthyllycaconitine (MLA) pour les récepteurs α₇ nicotiniques, la dihydro-β-érythroïdine pour les récepteurs α₄β₂ nicotiniques et le NE100 pour les récepteurs σ₁. Les effets des antagonistes ont été examinés sur les deux tests comportementaux d'alternance spontanée et d'évitement passif (Fig. 6). Il est apparu que la MLA a bloqué l'effet anti-amnésiant du composé **3b** à la dose de 3 mg/kg IP en alternance spontanée (Fig. 6a) et atténué l'effet à la dose de 1 mg/kg IP en évitement passif (Fig. 6b). Le DhβE est apparue inefficace aux deux doses testées en alternance spontanée (Fig. 6c) et en évitement passif (Fig. 6d). Le NE100, testé à la seule dose de 1 mg/kg IP, a bloqué l'effet du composé **3b** en alternance spontanée (Fig. 6e) et atténué l'effet en évitement passif (Fig. 6f). Ces résultats confirment que les effets anti-amnésiants, et possiblement neuroprotecteurs, du composé **3b** reposent sur des composantes α₇ nicotinique et σ₁.

En résumé, les résultats susmentionnés permettent d'établir les conclusions suivantes :
- sur le modèle non-transgénique de MA, induit par injection ICV de peptide Aβ₂₅₋₃₅ chez la souris, que le composé **3b** et le composé **3c** ont des effets neuroprotecteurs aux niveaux comportemental et biochimique. Les composés sont actifs à des doses faibles (0,7 et 0,3 mg/kg IP respectivement) du même ordre de grandeur que les molécules de référence dans ce modèle : 0,5 mg/kg IP pour le donepezil et 1 mg/kg IP pour la mémantine, par exemple. La récupération fonctionnelle est totale. L'ensemble des marqueurs biochimiques de toxicité examinés jusqu'ici dans le modèle, concernant le stress oxydant, l'apoptose, la neuroinflammation, les altérations synaptiques et du tonus cholinergique, sont atténués ou bloqués par le composé **3b** à sa dose active.
- une première étude pharmacologique suggère que les effets anti-amnésiants, et possiblement neuroprotecteurs, du composé **3b** reposent sur une composante α₇ nicotinique et une composante σ₁. Un profil de modulateur allostérique positif (PAM) sur ces deux récepteurs doit être recherché. Ces cibles sont susceptibles d'agir en synergie, ce qui expliquerait l'efficacité in vivo de ces composés.

### 2. Criblage in vivo des dérivés

A partir des molécules issues de la synthèse, un panel de 8 dérivés a été sélectionné et criblé pour leur potentielle efficacité neuroprotectrice. Les composés ont été injectés à la dose de 0,3 mg/kg IP, soit la dose maximale non-active du **3b** et comparés au **3b**, injecté à 0,3 et 0,7 mg/kg IP. Les composés ont été injectés immédiatement avant le peptide Aβ₂₅₋₃₅ ICV et les souris ont été testées en alternance spontanée au jour 7, en évitement passif aux jours 8 et 9 et le niveau de ROS mesuré dans l'hippocampe (Fig. 7). Pour chaque réponse (alternance spontanée : Fig. 7a, latence de passage en évitement passif : Fig. 7b, et niveau de fluorescence du DCF : Fig. 7c), un niveau 50% de l'effet entre les valeurs des animaux ScAβ et Aβ₂₅₋₃₅ a été pris comme critère d'efficacité. On a vérifié que la dose de **3b** de 0,7 mg/kg IP, mais pas 0,3, permettait d'atteindre ce critère. Les composés **3c**, **3n** et **3l** ont atteint le critère pour les 3 paramètres. Le composé **3c** est un isomère de position de l'atome de chlore du **3b.** Quant au composé **3n**, il possède un groupement 5-pyrimidyle attaché à l'atome de phosphore et pour le composé **3l** un groupement 4-pyridinyle. L'efficacité du **3c**, aussi efficace à 0,3 mg/kg IP que le **3b** à 0,7 mg/kg IP, a conduit à le sélectionner pour la suite des travaux.

### 3. Analyse de la protection induite par les phosphinolactones dans un modèle transgénique de la maladie

Les souris TG2576, surexprimant la protéine précurseur amyloïde humaine portant la double mutation suédoise, APP_{Swe}, développent la pathologie vers l'âge de 8-10 mois et jusqu'à 15-17 mois, âge auquel 95% des animaux montrent les signes comportementaux, biochimiques et morphologiques de la maladie. Le composé **3b** a été administré en traitement chronique, aux doses de 0,7 mg/kg/j IP puis de 2 mg/kg/j IP. L'effet du composé **3c**, à la dose de 1 mg/kg/j IP, a également été documenté.

Les animaux traités avec **3b** ont été analysés au test d'alternance spontanée après 1 et 2 mois de traitement (Fig. 8a). Après 1 mois de traitement, **3b** n'affecte pas les performances des animaux contrôles de souche sauvage (WT), mais les animaux APP_{Swe} n'alternent plus (Fig. 8a). Le traitement avec **3b**, à 2 mg/kg/j mais pas à 0,7 mg/kg/j, prévient significativement le déficit d'alternance. Après 2 mois de traitement, les animaux de la souche contrôle (WT) et APP_{Swe} montrent toujours une alternance correcte et un déficit, respectivement (Fig. 8b). Les deux doses de **3b** atténuent le déficit chez les souris APP_{Swe}, mais la dose la plus faible semble impacter l'alternance chez les animaux WT (Fig. 8b). L'analyse de la locomotion après 1 mois (Fig. 8c) ou 2 mois de traitement (Fig. 8d), ne montre pas de différence entre les groupes d'animaux.

Après 2 mois de traitement, les souris ont été examinées dans un test complexe d'apprentissage spatial à long-terme, la localisation d'une plate-forme invisible dans une piscine circulaire de 1,5 m de diamètre, puis au test de reconnaissance d'objet. Les profils d'acquisition en piscine sont présentés Fig. 9a,b. Chaque essai correspond à la valeur médiane de 3 nages par jour. Les animaux WT et WT traités avec **3b**, 2 mg/kg/j, ont acquis efficacement le principe du test et la localisation de la plate-forme (Fig. 9a). Les animaux APP_{Swe} ont montré également un profil qui diminue avec les essais, mais les latences sont significativement plus élevées que pour les animaux WT (Fig. 9b). Le traitement avec **3b** tend à diminuer ces latences. 48 h après la dernière session d'acquisition, la plate-forme est retirée de la piscine et le temps de présence dans chacun des quarts de la piscine analysé. Les résultats (Fig. 9c) présentent le temps dans le quart d'entrainement (où était placée la plate-forme) et la moyenne des autres quarts. Il apparaît que les animaux WT montrent une présence préférentielle dans le quart d'entrainement très significative. Les souris APP_{Swe} ne montrent pas de préférence spatiale, mais le traitement à 3b restaure de façon significative la présence préférentielle dans le quart d'entrainement (Fig. 9c). On note cependant une diminution des performances spatiales chez les souris WT traitées avec **3b** à 2 mg/kg/j IP, bien que la préférence spatiale persiste significativement.

Dans le test de reconnaissance d'objet, la motivation des animaux à aller se familiariser avec les objets a d'abord été analysée lors des sessions 2 et 3 du test (Fig. 9d). Il apparaît que les animaux APP_{Swe} montrent une diminution significative du nombre de contacts durant les sessions de 10 min. Cette diminution est prévenue par le traitement avec **3b**, sans effet chez les animaux WT (Fig. 9d). L'analyse de la reconnaissance des objets a donné des résultats mitigés en termes de nombre de contacts (Fig. 9e), mais plus clairs en termes de durée des contacts (Fig. 9f). Il apparait que si aucun des groupes ne montre de préférence durant la session 2, les groupes WT montrent bien une préférence pour le nouvel objet lors de la session 3 (Fig. 9f). Les animaux APP_{Swe} ne montrent pas de préférence d'objet. Ce déficit est atténué, mais de manière non-significative, par le traitement avec le comoosé **3b** (Fig. 9f).

Dans la série d'expérience avec le composé **3c**, à la dose de 1 mg/kg/j, les animaux ont également été analysés en alternance spontanée après 1 et 2 mois de traitement (Fig. 10). Après 1 mois de traitement, le déficit d'alternance observé chez les animaux APP_{Swe} est atténué par le composé **3c** (Fig. 10a). Après 2 mois, le déficit plus marqué chez les animaux APP_{Swe} est significativement bloqué par le traitement avec **3c** (Fig. 10b). Aucun effet sur la locomotion lors des sessions de labyrinthe en Y n'a été observé entre les groupes (Fig. 10c,d). Les animaux ont également été analysés après 2 mois de traitement dans le test d'apprentissage spatial en piscine et dans le test de reconnaissance d'objet (Fig. 11). Les animaux WT et WT traités avec **3c**, 1 mg/kg/j, ont acquis efficacement le principe du test et la localisation de la plate-forme (Fig. 11a). Les animaux APP_{Swe} ont également montré un profil similaire aux animaux WT, suggérant que le déficit mnésique est peu marqué dans ce groupe expérimental (Fig. 11b). Le traitement avec **3c** n'affecte pas le profil d'acquisition (Fig. 11b). Lors du test d'essai, 48 h après le dernier entrainement, les animaux WT montrent une présence préférentielle dans le quart d'entrainement très significative (Fig. 13c). Les souris APP_{Swe} montrent également une préférence spatiale mais moins marquée puisque la présence dans le quart d'entrainement n'est pas significativement différente du niveau de hasard (15 s). Le traitement avec le composé **3c** restaure de façon significative la présence préférentielle dans le quart d'entrainement (Fig. 11c). On note une diminution des performances spatiales chez les souris WT traitées avec 3c, bien que la préférence spatiale persiste significativement.

Dans le test de reconnaissance d'objet, l'analyse de la motivation des animaux à aller se familiariser avec les objets (Fig. 11d) a confirmé que les animaux APP_{Swe} montrent une diminution significative du nombre de contacts durant les sessions de 10 min. Cette diminution est bloquée par le traitement avec **3c**, sans effet chez les animaux WT (Fig. 11d). L'analyse de la reconnaissance des objets a donné des résultats cohérents, qu'ils soient traités en nombre de contacts (Fig. 11e) ou en durée des contacts (Fig. 11f). Il apparait qu'aucun des groupes ne montre de préférence significative durant la session 2, même si les valeurs des nombres de contact s'éloignent de 50% (Fig. 11e). Par contre, lors de la session 3, les groupes WT montrent bien une préférence pour le nouvel objet (Fig. 11e,f). Les animaux APP_{Swe} ne montrent pas de préférence d'objet. Ce déficit est significativement bloqué ou atténué par le traitement au CL420 (Fig. 11e,f).

Divers paramètres biochimiques ont été analysés dans l'hippocampe des souris APP_{Swe} suite aux traitements avec **3b**, 2 mg/kg/j IP, ou **3c**, 1 mg/kg/j IP (Fig. 12). Le traitement avec **3b** a diminué de façon significative le stress oxydant et le niveau de TNFα, mais n'a pas affecté les niveaux de synaptophysine, de Bax et d'IL1β (Fig. 12a). Le traitement avec **3c** a provoqué une diminution de l'expression de Bax, mais pas des niveaux de stress oxydant, de synaptophysine ou d'IL1β (Fig. 12b).

En résumé, les effets de 2 composés phosphinolactones, **3b**, la molécule tête de file, et **3c**, issu du criblage pharmacologique, ont été analysés en traitement chronique (2 mois) dans un modèle de souris transgénique de référence de la MA, les souris APP_{Swe}. Les doses utilisées pour ces composés, 1 ou 2 mg/kg/j IP, sont faibles et du même ordre de grandeur que celles utilisées pour les molécules de référence, donepezil ou mémantine, par exemple, dans ce modèle animal. Ces doses actives suggèrent que la biodisponibilité des molécules est très correcte et surtout, indiquent un mécanisme d'action particulièrement efficace compte-tenu du profil pharmacologique indéterminé à ce jour de ces molécules. Les composés sont efficaces pour prévenir les déficits d'apprentissage et de mémorisation observés chez les animaux APP_{Swe}, sur des tests classiques de cognition animale (alternance spontanée, apprentissage spatial en piscine et reconnaissance d'objet). Les résultats biochimiques sont plus parcellaires et s'ils suggèrent que les deux traitements ne sont pas que symptomatiques, des études plus approfondies restent nécessaires pour établir un effet neuroprotecteur potentiel.

### 4. Modulation de la protéine sigma-1

Les résultats de la Figure 13 montrent que :
- Le composé 3b n'entre pas en compétition avec le radiotraceur sigma-1 sur des préparations de membranes de cerveau de cobaye et donc que la molécule ne se fixe pas sur la protéine sigma-1 de façon orthostatique.
- Le composé 3b est antidépresseur sur le test de nage forcée et cet effet est bloqué par une antagoniste pharmacologique sélectif des récepteurs sigma-1 et chez les souris KO pour le récepteur sigma-1. La molécule présente des effets comportementaux de type sigma-1.
- A une dose où la molécule n'est pas active (5 mg/kg), combinée à une dose où l'igmésine (agoniste sigma-1 historique) elle-même n'est pas active (10 mg/kg), on observe un effet antidépresseur significatif. Cet effet est entièrement bloqué par un antagoniste sélectif sigma-1.

En conclusion, le composé 3b se comporte comme un modulateur allostérique (PAM) sigma-1.

## Revendications

1. Composé de formule (I) suivante : 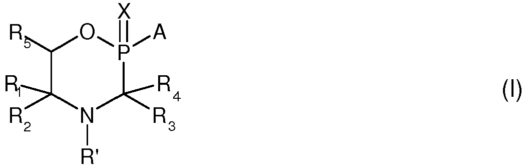 dans laquelle :
- X est O ou S ;
- A est choisi dans le groupe constitué :
• des groupes (C₆-C₁₀)aryles,
• des groupes hétéroaryles comprenant de 5 à 10 atomes, et
• des hétérocycloalkyles comprenant de 5 à 10 atomes,
lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ;
Rₐ et R_{b}, identiques ou différents, étant H ou un groupe (C₁-C₆)alkyle ;
R_{C} étant un radical -(C₁-C₆)alkylène-(C₆-C₁₀)aryle, notamment un radical -CH₂-(C₆-C₁₀)aryle ;
R'ₐ étant choisi parmi les groupes CF₃, CHF₂ et CH₂F ;
- R' est H ou un groupe (C₁-C₆)alkyle ;
- R₁, R₂, R₃, R₄ et R₅, identiques ou différents, sont choisis dans le groupe constitué de :
- H,
- des groupes (C₁-C₆)alkyles, et
- des groupes (C₆-C₁₀)aryles,
R₁ et R₂ pouvant former ensemble, avec l'atome de carbone qui les porte, un cycle spiranique comprenant de 3 à 6 atomes de carbone, et/ou
R₃ et R₄ pouvant former ensemble, avec l'atome de carbone qui les porte, un cycle spiranique comprenant de 3 à 6 atomes de carbone,
pour son utilisation pour le traitement de maladies neurodégénératives.

2. Composé pour son utilisation selon la revendication 1, dans lequel R' est H.

3. Composé pour son utilisation selon la revendication 1 ou 2, dans lequel R₁, R₂, R₃ et R₄, identiques ou différents, sont des groupes (C₁-C₆)alkyles et R₅ est H.

4. Composé pour son utilisation selon l'une quelconque des revendications 1 à 3, dans lequel X est O.

5. Composé pour son utilisation selon l'une quelconque des revendications 1 à 4, dans lequel A est un groupe phényle, éventuellement substitué.

6. Composé pour son utilisation selon l'une quelconque des revendications 1 à 5, dans lequel A est un hétéroaryle comprenant 6 atomes dont au moins un atome d'azote.

7. Composé pour son utilisation selon la revendication 6, dans lequel A est choisi dans le groupe constitué des groupes 2-pyridinyle, 3-pyridinyle, 4-pyridinyle, 2-pyrimidinyle et 5-pyrimidinyle.

8. Composé de formule (I-1) suivante : 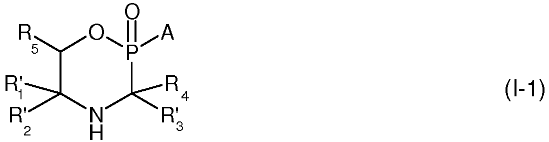 dans laquelle :
- R'₁, R'₂ et R'₃, identiques ou différents, sont des groupes (C₁-C₆)alkyles ou des groupes (C₆-C₁₀)aryles, R'₁, R'₂ et R'₃ étant de préférence des groupes méthyle,
- R₄ et R₅, identiques ou différents, sont choisis dans le groupe constitué de :
- H,
- des groupes (C₁-C₆)alkyles, et
- des groupes (C₆-C₁₀)aryles,
R'₁ et R'₂ pouvant former ensemble, avec l'atome de carbone qui les porte, un cycle spiranique comprenant de 3 à 6 atomes de carbone, et/ou R'₃ et R₄ pouvant former ensemble, avec l'atome de carbone qui les porte, un cycle spiranique comprenant de 3 à 6 atomes de carbone,
- A est choisi dans le groupe constitué :
• des groupes (C₆-C₁₀)aryles,
• des groupes hétéroaryles comprenant de 5 à 10 atomes, et
• des hétérocycloalkyles comprenant de 5 à 10 atomes,
lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ ;
Rₐ et R_{b}, identiques ou différents, étant H ou un groupe (C₁-C₆)alkyle ;
R_{c} étant un radical -(C₁-C₆)alkylène-(C₆-C₁₀)aryle, notamment un radical -CH₂-(C₆-C₁₀)aryle ;
R'ₐ étant choisi parmi les groupes CF₃, CHF₂ et CH₂F ;
et, lorsque R'₁=R'₂=R'₃=Me, R₄=Me ou H et R₅=H, A est différent des groupes suivants :

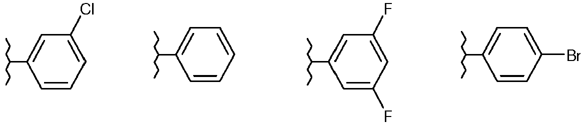

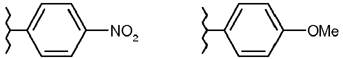

9. Composé selon la revendication 8, dans laquelle A est un cycle aromatique comprenant 6 atomes, dont au moins un atome d'azote, ou A est choisi parmi les groupes suivants :

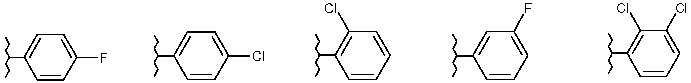

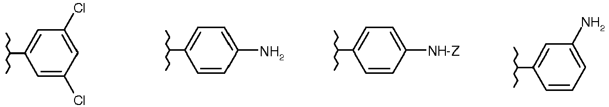

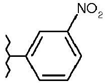

10. Composé de formule (I-2) suivante : 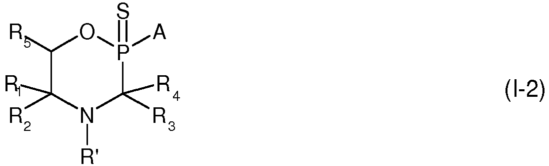 dans laquelle :
- A est choisi dans le groupe constitué :
• des groupes (C₆-C₁₀)aryles,
• des groupes hétéroaryles comprenant de 5 à 10 atomes, et
• des hétérocycloalkyles comprenant de 5 à 10 atomes,
lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ ;
Rₐ et R_{b}, identiques ou différents, étant H ou un groupe (C₁-C₆)alkyle ;
R_{C} étant un radical -(C₁-C₆)alkylène-(C₆-C₁₀)aryle, notamment un radical -CH₂-(C₆-C₁₀)aryle ;
R'ₐ étant choisi parmi les groupes CF₃, CHF₂ et CH₂F ;
- R' est H ou un groupe (C₁-C₆)alkyle ;
- R₁, R₂, R₃, R₄ et R₅, identiques ou différents, sont choisis dans le groupe constitué de :
- H,
- des groupes (C₁-C₆)alkyles, et
- des groupes (C₆-C₁₀)aryles,
R₁ et R₂ pouvant former ensemble, avec l'atome de carbone qui les porte, un cycle spiranique comprenant de 3 à 6 atomes de carbone, et/ou R₃ et R₄ pouvant former ensemble, avec l'atome de carbone qui les porte, un cycle spiranique comprenant de 3 à 6 atomes de carbone.

11. Composé de formule (I-3) suivante : 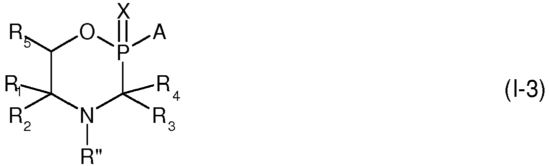 dans laquelle :
- X est O ou S ;
- A est choisi dans le groupe constitué :
• des groupes (C₆-C₁₀)aryles,
• des groupes hétéroaryles comprenant de 5 à 10 atomes, et
• des hétérocycloalkyles comprenant de 5 à 10 atomes,
lesdits groupes aryles, hétéroaryles et hétérocycloalkyles étant éventuellement substitués par au moins un substituant choisi dans le groupe constitué des atomes d'halogène, ORₐ, SRₐ, NO₂, NRₐR_{b}, N(Rₐ)COOR_{c}, R'ₐ et OR'ₐ ;
Rₐ et R_{b}, identiques ou différents, étant H ou un groupe (C₁-C₆)alkyle ;
R_{c} étant un radical -(C₁-C₆)alkylène-(C₆-C₁₀)aryle, notamment un radical -CH₂-(C₆-C₁₀)aryle ;
R'ₐ étant choisi parmi les groupes CF₃, CHF₂ et CH₂F ;
- R" est un groupe (C₁-C₆)alkyle ;
- R₁, R₂, R₃, R₄ et R₅, identiques ou différents, sont choisis dans le groupe constitué de :
- H,
- des groupes (C₁-C₆)alkyles, et
- des groupes (C₆-C₁₀)aryles,
R₁ et R₂ pouvant former ensemble, avec l'atome de carbone qui les porte, un cycle spiranique comprenant de 3 à 6 atomes de carbone, et/ou R₃ et R₄ pouvant former ensemble, avec l'atome de carbone qui les porte, un cycle spiranique comprenant de 3 à 6 atomes de carbone.

12. Composition pharmaceutique, comprenant au moins un composé selon l'une quelconque des revendications 8 à 11, en association avec au moins un véhicule pharmaceutiquement acceptable.

## Patentansprüche

1. Verbindung der folgenden Formel (I): 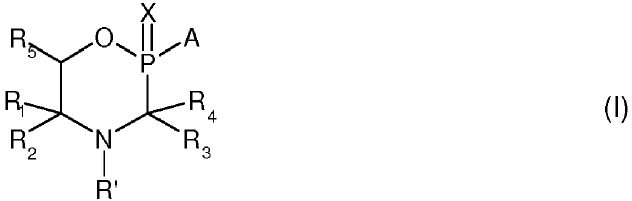 in der:
- X O oder S ist;
- A ausgewählt ist aus der Gruppe, gebildet aus:
• (C₆-C₁₀)-Arylgruppen,
• Heteroarylgruppen, die 5 bis 10 Atome enthalten, und
• Heterozykloalkyle, die 5 bis 10 Atome enthalten,
wobei die Arylgruppen, Heteroarylgruppen und Heterozykloalkyle gegebenenfalls durch mindestens einen Substituenten substituiert sind, ausgewählt aus der Gruppe, bestehend aus Halogenatomen, ORₐ, SRₐ, NO₂, NRₐR_{b}, N(Rₐ)COOR_{c}, R'ₐ und OR'ₐ;
Rₐ und R_{b}, identisch oder unterschiedlich, H oder eine (C₁-C₆)-Alkylgruppe ist;
R_{c} ein Radikal -(C₁-C₆)-Alkylen-(C₆-C₁₀)-Aryl, insbesondere ein Radikal -CH₂-(C₆-C₁₀)-Aryl ist;
R'ₐ ausgewählt ist aus den Gruppen CF₃, CHF₂ und CH₂F;
- R' H oder eine (C₁-C₆)-Alkylgruppe ist;
- R₁, R₂, R₃, R₄ und R₅, identisch oder unterschiedlich, ausgewählt ist aus der Gruppe, bestehend aus:
- H,
- (C₁-C₆)-Alkylgruppen und
- (C₆-C₁₀)-Arylgruppen,
wobei R₁ und R₂ zusammen mit dem Kohlenstoffatom, das sie trägt, einen Spirozyklus bilden können, der 3 bis 6 Kohlenstoffatome enthält, und/oder
R₃ und R₄ zusammen mit dem Kohlenstoffatom, das sie trägt, einen Spirozyklus bilden können, der 3 bis 6 Kohlenstoffatome enthält,
für ihre Verwendung für die Behandlung von neurodegenerativen Erkrankungen.

2. Verbindung für ihre Verwendung nach Anspruch 1, bei der R' H ist.

3. Verbindung für ihre Verwendung nach Anspruch 1 oder 2, bei der R₁, R₂, R₃ und R₄, identisch oder unterschiedlich, (C₁-C₆)-Alkylgruppen sind und R₅ H ist.

4. Verbindung für ihre Verwendung nach einem beliebigen der Ansprüche 1 bis 3, bei der X O ist.

5. Verbindung für ihre Verwendung nach einem beliebigen der Ansprüche 1 bis 4, bei der A eine Phenylgruppe, gegebenenfalls substituiert, ist.

6. Verbindung für ihre Verwendung nach einem beliebigen der Ansprüche 1 bis 5, bei der A ein Heteroaryl ist, das 6 Atome enthält, von denen mindestens ein Atom Stickstoff ist.

7. Verbindung für ihre Verwendung nach Anspruch 6, bei der A ausgewählt ist aus der Gruppe, bestehend aus 2-Pyridinyl-, 3-Pyridinyl-; 4-Pyridinyl-, 2-Pyrimidinyl- und 5-Pyrimidinylgruppen.

8. Verbindung der folgenden Formel (I-1): 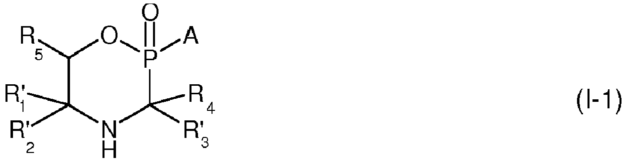 bei der:
- R'₁, R'₂ und R'₃, identisch oder unterschiedlich, (C₁-C₆)-Alkylgruppen oder (C₆-C₁₀)-Arylgruppen sind, wobei R'₁, R'₂ und R'₃ vorzugsweise Methylgruppen sind,
- R₄ und R₅, identisch oder unterschiedlich, ausgewählt sind aus der Gruppe, bestehend aus:
- H,
- (C₁-C₆)-Alkylgruppen und
- (C₆-C₁₀)-Arylgruppen,
wobei R'₁ und R'₂ zusammen mit dem Kohlenstoffatom, das sie trägt, einen Spirozyklus bilden kann, der 3 bis 6 Kohlenstoffatome umfasst, und/oder
R'₃ und R₄ zusammen mit dem Kohlenstoffatom, das sie trägt, einen Spirozyklus bilden kann, der 3 bis 6 Kohlenstoffatome enthält,
- A ausgewählt ist aus der Gruppe, bestehend aus:
• (C₆-C₁₀)-Arylgruppen,
• Heteroarylgruppen, die 5 bis 10 Atome enthalten, und
• Heterozykloalkyle, die 5 bis 10 Atome enthalten,
wobei die Arylgruppen, Heteroarylgruppen und Heterozykloalkyle gegebenenfalls durch mindestens einen Substituenten substituiert sind, der ausgewählt aus der Gruppe, bestehend aus Halogenatomen, ORₐ, SRₐ, NO₂, NRₐR_{b}, N(Rₐ)COOR_{c}, R'ₐ und OR'ₐ;
Rₐ und R_{b}, identisch oder Unterschiedlich, H oder eine (C₁-C₆)-Alkylgruppe ist;
R_{c} ein Radikal -(C₁-C₆)-Alkylen-(C₆-C₁₀)-Aryl, insbesondere ein Radikal -CH₂-(C₆-C₁₀)-Aryl ist;
R'ₐ ausgewählt ist aus den Gruppen CF₃, CHF₂ und CH₂F;
und, wenn R'₁ = R'₂ = R'₃ = Me, R₄ = Me oder H und R₅ = H ist, ist A unterschiedlich zu den folgenden Gruppen:

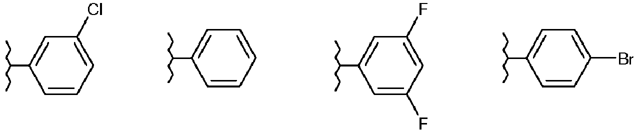

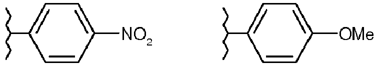

9. Verbindung nach Anspruch 8, bei der A ein aromatischer Ring ist, der 6 Atome enthält, von denen mindestens eines ein Stickstoffatom ist, oder A ausgewählt ist aus den folgenden Gruppen:

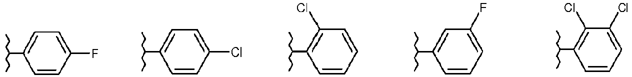

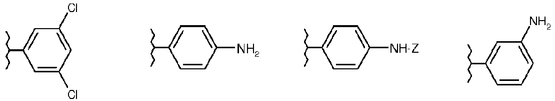

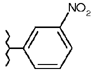

10. Verbindung der folgenden Formel (I-2): 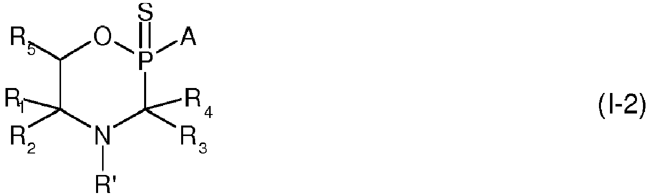 bei der:
- A ausgewählt ist aus der Gruppe, bestehend aus:
• (C₆-C₁₀)-Arylgruppen,
• Heteroarylgruppen, die 5 bis 10 Atome enthalten, und
• Heterozykloalkyle, die 5 bis 10 Atome enthalten,
wobei die Arylgruppen, Heteroarylgruppen und Heterozykloalkyle gegebenenfalls durch mindestens einen Substituenten substituiert sind, der ausgewählt aus der Gruppe, bestehend aus Halogenatomen, ORₐ, SRₐ, NO₂, NRₐR_{b}, N(Rₐ)COOR_{c}, R'ₐ und OR'ₐ;
Rₐ und R_{b}, identisch oder unterschiedlich, H oder eine (C₁-C₆)-Alkylgruppe ist;
R_{c} ein Radikal -(C₁-C₆)-Alkylen-(C₆-C₁₀)-Aryl, insbesondere ein Radikal -CH₂-(C₆-C₁₀)-Aryl ist;
R'ₐ ausgewählt ist aus den Gruppen CF₃, CHF₂ und CH₂F;
- R' H oder eine (C₁-C₆)-Alkylgruppe ist;
- R₁, R₂, R₃, R₄ und R₅, identisch oder unterschiedlich, ausgewählt ist aus der Gruppe, bestehend aus:
- H,
- (C₁-C₆)-Alkylgruppen und
- (C₆-C₁₀)-Arylgruppen,
wobei R₁ und R₂ zusammen mit dem Kohlenstoffatom, das sie trägt, einen Spirozyklus bilden können, der 3 bis 6 Kohlenstoffatome enthält, und/oder
R₃ und R₄ zusammen mit dem Kohlenstoffatom, das sie trägt, einen Spirozyklus bilden können, der 3 bis 6 Kohlenstoffatome enthält.

11. Verbindung der folgenden Formel (I-3): 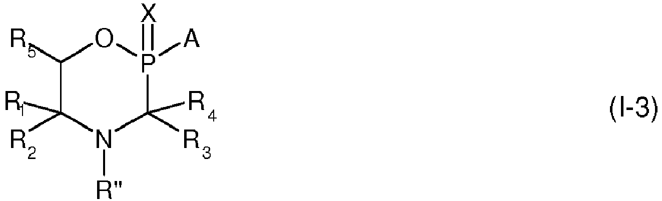 bei der:
- X O oder S ist,
- A ausgewählt aus der Gruppe ist, gebildet aus:
• (C₆-C₁₀)-Arylgruppen,
• Heteroarylgruppen, die 5 bis 10 Atome enthalten, und
• Heterozykloalkyle, die 5 bis 10 Atome enthalten,
wobei die Arylgruppen, Heteroarylgruppen und Heterozykloalkyle gegebenenfalls durch mindestens einen Substituenten substituiert sind, der ausgewählt aus der Gruppe, bestehend aus Halogenatomen, ORₐ, SRₐ, NO₂, NRₐR_{b}, N(Rₐ)COOR_{c}, R'ₐ und OR'ₐ;
Rₐ und R_{b}, identisch oder unterschiedlich, H oder eine (C₁-C₆)-Alkylgruppe ist;
R_{c} ein Radikal -(C₁-C₆)-Alkylen-(C₆-C₁₀)-Aryl, insbesondere ein Radikal -CH₂-(C₆-C₁₀)-Aryl ist;
R'ₐ ausgewählt ist aus den Gruppen CF₃, CHF₂ und CH₂F;
- R" eine (C₁-C₆)-Alkylgruppe ist;
- R₁, R₂, R₃, R₄ und R₅, identisch oder unterschiedlich, ausgewählt ist aus der Gruppe, bestehend aus:
- H,
- (C₁-C₆)-Alkylgruppen und
- (C₆-C₁₀)-Arylgruppen,
wobei R₁ und R₂ zusammen mit dem Kohlenstoffatom, das sie trägt, einen Spirozyklus bilden können, der 3 bis 6 Kohlenstoffatome enthält, und/oder
R₃ und R₄ zusammen mit dem Kohlenstoffatom, das sie trägt, einen Spirozyklus bilden können, der 3 bis 6 Kohlenstoffatome enthält.

12. Pharmazeutische Zusammensetzung, mindestens eine Verbindung nach einem beliebigen der Ansprüche 8 bis 11 zusammen mit mindestens einem pharmazeutisch verträglichen Träger enthaltend.

## Claims

1. Compound of following formula (I): 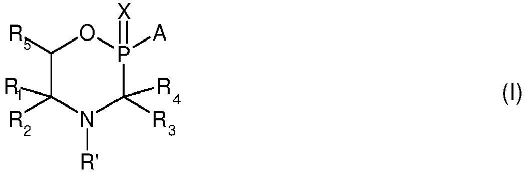 where:
- X is O or S;
- A is selected from the group formed by:
• (C₆-C₁₀)aryl groups,
• heteroaryl groups having 5 to 10 atoms, and
• heterocycloalkyls having 5 to 10 atoms,
said aryl, heteroaryl and heterocycloalkyl groups optionally being substituted by at least one substituent selected from the group formed by halogen atoms, ORₐ, SRₐ, NO₂, NRₐR_{b}, N(Rₐ)COOR_{c}, R'ₐ and OR'ₐ;
Rₐ and R_{b}, the same or different, being H or (C₁-C₆)alkyl group;
R_{c} being a -(C₁-C₆)alkylene-(C₆-C₁₀)aryl radical, in particular a -CH₂-(C₆-C₁₀)aryl radical;
R'ₐ being selected from the groups CF₃, CHF₂ and CH₂F
- R' is H or (C₁-C₆)alkyl group;
- R₁, R₂, R₃, R₄ and R₅, the same or different, are selected from the group formed by:
- H,
- (C₁-C₆)alkyl groups, and
- (C₆-C₁₀)aryl groups,
R₁ and R₂, together with their carrier carbon atom, able to form a spiran ring having 3 to 6 carbon atoms, and/or
R₃ and R₄, together with their carrier carbon atom, able to form a spiran ring having 3 to 6 carbon atoms.
for use thereof in the treatment of neurodegenerative diseases.

2. Compound for use thereof according claim 1, wherein R' is H.

3. Compound for use thereof according to claim 1 or 2, wherein R₁, R₂, R₃ and R₄, the same or different, are (C₁-C₆)alkyl groups and R₅ is H.

4. Compound for use thereof according to any of claims 1 to 3, wherein X is O.

5. Compound for use thereof according to any of claims 1 to 4, wherein A is an optionally substituted phenyl group.

6. Compound for use thereof according to any of claims 1 to 5, wherein A is a heteroaryl having 6 atoms of which at least one is a nitrogen atom.

7. Compound for use thereof according to claim 6, wherein A is selected from the group formed by 2-pyridinyl, 3-pyridinyl, 4-pyridinyl, 2-pyrimidinyl and 5-pyrimidinyl groups.

8. Compound of following formula (I-1): 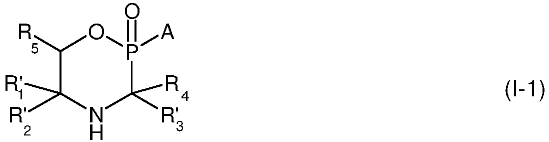 where:
- R'₁, R'₂ and R'₃, the same or different, are (C₁-C₆)alkyl groups or (C₆-C₁₀)aryl groups, R'₁, R'₂ and R'₃ preferably being methyl groups.
- R₄ and R₅, the same or different, are selected from the group formed by:
- H,
- (C₁-C₆)alkyl groups, and
- (C₆-C₁₀)aryl groups,
R'₁ and R'₂, together with their carrier carbon atom, able to form a spiran ring having 3 to 6 carbon atoms, and/or
R'₃ and R₄, together with their carrier carbon atom, able to form a spiran ring having 3 to 6 carbon atoms.
- A is selected from the group formed by:
• (C₆-C₁₀)aryl groups,
• heteroaryl groups having 5 to 10 atoms, and
• heterocycloalkyls having 5 to 10 atoms,
said aryl, heteroaryl and heterocycloalkyl groups optionally being substituted by at least one substituent selected from the group formed by halogen atoms, ORₐ, SRₐ, NO₂, NRₐR_{b}, N(Rₐ)COOR_{c}, R'ₐ and OR'ₐ;
Rₐ and R_{b}, the same or different, being H or (C₁-C₆)alkyl group;
R_{c} being a -(C₁-C₆)alkylene-(C₆-C₁₀)aryl radical, in particular a -CH₂-(C₆-C₁₀)aryl radical;
R'ₐ being selected from among the groups CF₃, CHF₂ and CH₂F;
and, when R'₁=R'₂=R'₃=Me, R₄=Me or H and R₅=H, A differs from the following groups:

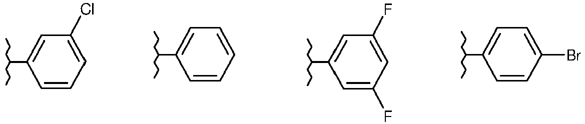

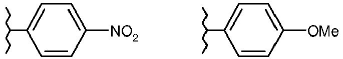

9. The compound according to claim 8, wherein A is an aromatic ring having 6 atoms, at least one of which is a nitrogen atom, or A is selected from among the following groups:

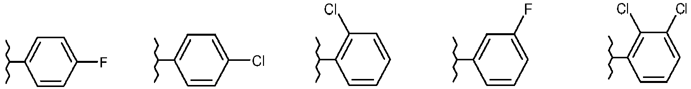

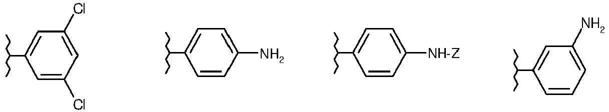

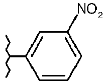

10. Compound of following formula (I-2): 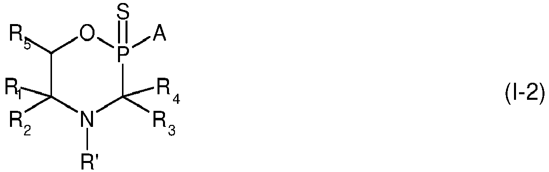 where:
- A is selected from the group formed by:
• (C₆-C₁₀)aryl groups,
• heteroaryl groups having 5 to 10 atoms, and,
• heterocycloalkyls having 5 to 10 atoms,
said aryl, heteroaryl and heterocycloalkyl groups optionally being substituted by at least one substituent selected from the group formed by halogen atoms, ORₐ, SRₐ, NO₂, NRₐR_{b}, N(Rₐ)COOR_{c}, R'ₐ and OR'ₐ;
Rₐ and R_{b}, the same or different, being H or (C₁-C₆)alkyl group;
R_{c} being a -(C₁-C₆)alkylene-(C₆-C₁₀)aryl radical, in particular a -CH₂-(C₆-C₁₀)aryl radical;
R'ₐ being selected from the groups CF₃, CHF₂ and CH₂F;
- R' is H or (C₁-C₆)alkyl group;
- R₁, R₂, R₃, R₄ and R₅, the same or different, are selected from the group formed by:
- H,
- (C₁-C₆)alky groups, and
- (C₆-C₁₀)aryl groups,
R₁ and R₂, together with their carrier carbon atom, able to form a spiran ring having 3 to 6 carbon atoms, and/or
R₃ and R₄, together with their carrier carbon atom, able to form a spiran ring having 3 to 6 carbon atoms.

11. Compound of following formula (I-3): 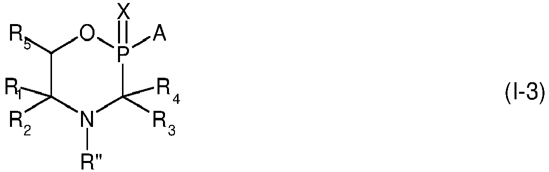 where:
- X is O or S;
- A is selected from the group formed by:
• (C₆-C₁₀)aryl groups,
• heteroaryl groups having 5 to 10 atoms, and
• heterocycloalkyls having 5 to 10 atoms,
said aryl, heteroaryl and heterocycloalkyl groups optionally being substituted by at least one substituent selected from the group formed by halogen atoms, ORₐ, SRₐ, NO₂, NRₐR_{b}, N(Rₐ)COOR_{c}, R'ₐ and OR'ₐ;
Rₐ and R_{b}, the same or different, being H or (C₁-C₆)alkyl group;
R_{c} being a -(C₁-C₆)alkylene-(C₆-C₁₀)aryl radical, in particular a -CH₂-(C₆-C₁₀)aryl radical;
R'ₐ being selected from the groups CF₃, CHF₂ and CH₂F;
- R" is a (C₁-C₆)alkyl group;
- R₁, R₂, R₃, R₄ and R₅, the same or different, are selected from the group formed by:
- H,
- (C₁-C₆)alky groups, and
- (C₆-C₁₀)aryl groups,
R₁ and R₂, together with their carrier carbon atom, able to form a spiran ring having 3 to 6 carbon atoms, and/or
R₃ and R₄, together with their carrier carbon atom, able to form a spiran ring having 3 to 6 carbon atoms.

12. Pharmaceutical composition comprising at least one compound according to any of claims 8 to 11, in association with at least one pharmaceutically acceptable vehicle.
